# EUROPEAN PATENT APPLICATION

(11) **EP 3 312 293 A1**
(43) Date of publication of application: **25.04.2018**
(21) Application number: 16811215.9
(22) Date of filing: 08.06.2016
(51) Int. Cl.: C12Q 1/68, C12M 1/34, C12N 15/09, G01N 33/53

(54) **METHOD FOR EXAMINING MICROORGANISM, KIT FOR EXAMINING MICROORGANISM, MICROARRAY FOR EXAMINING MICROORGANISM, CARRIER FOR DETECTING MOLD, METHOD FOR DETECTING MOLD AND KIT FOR DETECTING MOLD**

(30) Priority: 17.06.2015 JP 2015121906; 04.08.2015 JP 2015154195
(71) Applicant: Toyo Seikan Group Holdings, Ltd., Shinagawa-ku Tokyo 141-8627 (JP)
(72) Inventor: ISSHIKI, Atsunori, Yokohama-shi Kanagawa 240-0062 (JP)
(74) Representative: Schmitz, Joseph
(86) International application number: PCT/JP2016/002780
(87) International publication number: WO 2016/203740

(57) **Abstract**

To enable appropriate detection of microorganisms belonging to the genera Plasmodiophora, Pythium and Phytophthora. The present invention is directed to a method for detecting microorganism which comprises collecting a sample from a plant tissue, soil, water or any other environment, extracting DNA from microorganisms contained in the sample, conducting PCR with the use of the extracted DNA, and determining the presence or absence of microorganisms in the sample on the basis of the obtained amplification product, wherein the PCR is conducted, for a β-tubulin gene in DNA of microorganisms belonging to the genera Plasmodiophora, Pythium and Phytophthora as PCR amplification targets, with the use of the extracted DNA and a primer set which consists of a forward primer comprising at least one of base sequences represented by SEQ ID NOS: 1-3 and a reverse primer comprising at least one of base sequences represented by SEQ ID NOS: 4 and 5, and then the presence or absence of these microorganisms in the sample is determined on the basis of the obtained amplification product.

SEQUENCE LISTING

<110>  TOYO SEIKAN GROUP HOLDINGS, LTD.

<120>  METHOD, KIT AND MICROARRAY FOR TESTING MICROORGANISM, CARRIER FOR
DETECTING FUNGI, METHOD FOR DETECTING FUNGI, AND KIT
       FOR DETECTING FUNGI

<130>  TSK-1582-PCT

<160>  31

<170>

<210>  1
<211>  17
<212>  DNA
<213>  Artificial

<220>
<223>  synthetic DNA

<400>  1
aacaactggg ccaaggg                                                    17


<210>  2
<211>  23
<212>  DNA
<213>  Artificial

<220>
<223>  synthetic DNA

<400>  2
gctggtaaca actgggccaa ggg                                             23


<210>  3
<211>  23
<212>  DNA
<213>  Artificial

<220>
<223>  synthetic DNA

<400>  3
gccggcaaca actgggccaa ggg                                             23


<210>  4
<211>  23
<212>  DNA
<213>  Artificial

<220>
<223>  synthetic DNA

<400>  4
gtacccatac cggaaccggt acc                                             23


<210>  5

<211>  23
<212>  DNA
<213>  Artificial

<220>
<223>  synthetic DNA

<400>  5
gtacccatgc cggaaccagt acc                                               23


<210>  6
<211>  24
<212>  DNA
<213>  Artificial

<220>
<223>  synthetic DNA

<400>  6
atcctagccg aaacacaact aaag                                              24


<210>  7
<211>  24
<212>  DNA
<213>  Artificial

<220>
<223>  synthetic DNA

<400>  7
gtgagtcatc gaaattttga acgc                                              24


<210>  8
<211>  17
<212>  DNA
<213>  Artificial

<220>
<223>  synthetic DNA

<400>  8
gcgaactgcg atacgta                                                      17


<210>  9
<211>  24
<212>  DNA
<213>  Artificial

<220>
<223>  synthetic DNA

<400>  9
ttcgcactac gtatcgcagt tcgc                                              24


<210>  10
<211>  16
<212>  DNA
<213>  Artificial

<220>
<223>  synthetic DNA

<400>  10
agacacttca catctg                                                        16


<210>  11
<211>  24
<212>  DNA
<213>  Artificial

<220>
<223>  synthetic DNA

<400>  11
accatgcgag acacttcaca tctg                                               24


<210>  12
<211>  22
<212>  DNA
<213>  Plasmodiophora sp.

<400>  12
gactcagtcc tcgacaccgt cc                                                 22


<210>  13
<211>  37
<212>  DNA
<213>  Pythium sp.

<400>  13
cgacatccaa gaccgagtcg atcaattcag cgccttc                                 37


<210>  14
<211>  34
<212>  DNA
<213>  Pythium sp.

<400>  14
tgatctggaa gccctggaga cagtcacagc cctc                                    34


<210>  15
<211>  22
<212>  DNA
<213>  Phytophthora sp.

<400>  15
gactcggtgc ttgacgtcgt tc                                                 22


<210>  16
<211>  22
<212>  DNA
<213>  Phytophthora sp.

<400>  16
gaacgacgtc aagcaccgag tc                                                 22

<210>  17
<211>  34
<212>  DNA
<213>  Plasmodiophora sp. Pythium sp.Phytophthora sp.

<400>  17
gagggctgtg actgtctcca gggcttccag atca                                    34


<210>  18
<211>  40
<212>  DNA
<213>  Plasmodiophora sp.

<400>  18
tatccattgc taagagttgt aacatgtatg tatgtatgga                              40


<210>  19
<211>  35
<212>  DNA
<213>  Pythium sp.

<400>  19
acattccatc cctgactaca cagaaaaaag aaagg                                   35


<210>  20
<211>  33
<212>  DNA
<213>  Pythium sp. Phytophthora sp.

<400>  20
atattgcact ttcgggttat gcctggaagt atg                                     33


<210>  21
<211>  30
<212>  DNA
<213>  Pythium sp. Phytophthora sp.

<400>  21
cctggaagta tgtctgtatc agtgtccgta                                         30


<210>  101
<211>  46
<212>  DNA
<213>  Colletotrichum acutatum

<400>  101
gaagcctctc gcgggcctcc cctcccggcg ccggccccca ccacgg                       46


<210>  102
<211>  50
<212>  DNA
<213>  Colletotrichum acutatum

<400>  102
ggtcagtatg agatcgtgtc aaatgtcgaa atcgccgcgt cgctggcggt                   50

<210>  103
<211>  33
<212>  DNA
<213>  Fusarium solani

<400>  103
taatgttttt ctgagtaaac aagcaaataa att                                    33


<210>  104
<211>  40
<212>  DNA
<213>  Fusarium solani

<400>  104
attgcccgaa gcctatacca aatctagctg acatctgtag                             40


<210>  105
<211>  30
<212>  DNA
<213>  Alternaria solani

<400>  105
ggccaccccg ggaggtgcca gcccgccttc                                        30


<210>  106
<211>  55
<212>  DNA
<213>  Rhizoctonia solani

<400>  106
ttggggaatt tatttgttat tttttgtaat aaaatgataa taagtcattg aaccc            55


<210>  107
<211>  26
<212>  DNA
<213>  Artificial

<220>
<223>  synthetic DNA

<400>  107
ttggtcattt agaggaagta aaagtc                                            26


<210>  108
<211>  19
<212>  DNA
<213>  Artificial

<220>
<223>  synthetic DNA

<400>  108
ctgcgttctt catcgatgc                                                    19


<210>  109

<211>  24
<212>  DNA
<213>  Artificial

<220>
<223>  synthetic DNA

<400>  109
ggtaaccaaa tcggtgctgc tttc                                              24


<210>  110
<211>  24
<212>  DNA
<213>  Artificial

<220>
<223>  synthetic DNA

<400>  110
accctcagtg tagtgaccct tggc                                              24

## Description

### Technical Field

The present invention relates to a gene detection technology to identify the type of organisms etc. based on gene sequence. In particular, the present invention relates to a method for detecting microorganism, a kit for detecting microorganism and a microarray for detecting microorganism for detecting plant pathogens associated with soil-borne diseases.

Further, the present invention relates to a carrier for detecting fungi that is a pathogen of plant diseases, a method for detecting fungi and a kit for detecting fungi.

### Background Art

In recent years, in food inspection, environmental testing, etc., in order to specify the type of microorganisms such as fungi and food poisoning bacteria present in foods and the environment, DNA in a target gene is amplified by nucleic acid amplification method such as PCR (polymerase chain reaction) method, etc. and thereafter, by detecting the obtained amplification product, the type of the microorganism is determined.

Meanwhile, some plant pathogens associated with soil-borne diseases present in plant tissues, soil and water belong to different kingdoms from those to be detected in food inspections, environmental tests and the like. Even when PCR was performed using primers of these microorganisms as a detection target, it was difficult to amplify the DNA of soil-borne pathogenic fungi.

Also, in recent years, it has become important to confirm whether or not microorganisms such as fungi exist in food production sites, clinical sites, agricultural sites, cultural property protection environments, etc. to check safety and soundness, and to prevent their breeding.

In such fungi detection, generally, a morphological observation method (cultivation method) is conducted in which samples are collected from the environment and pre-cultured, then cultured for about 20 days in an optimal medium for each type of fungi, and then morphological features are observed, thereby to identify fungi (see Patent Document 3).

However, in this method, since it is necessary to carry out separate culturing for each type of fungi, there is a problem that the detection process becomes complicated. In addition, since it takes a long time to culture, there is a problem that it is inappropriate for examination requiring promptness such as inspection of indoors where human lives, inspection of food, diagnosis of plant diseases at agricultural site, etc. Furthermore, there is a problem that identification cannot be performed unless spores expressing morphological features are formed, which may result in waste of labor.

Recently, identification methods using genes are also carried out in detection of fungi. For example, after culturing fungi collected from the environment, DNA is extracted from the cultured fungi, the target region is amplified by PCR method or the like, and the amplification product is analyzed to identify fungi in the environment. As a method of analyzing the amplification product, a method of analyzing the size of the amplification product by electrophoresis, a method of using a DNA chip on which a probe that is complementarily coupled with the amplification product is immobilized and the like have been proposed (Patent Document 4 and 5).

Patent Document 1: JP-A-H10-234399
Patent Document 2: Japan Patent No. 5522820
Patent Document 3: JP-A-2007-195454
Patent Document 4: Japan Patent No. 5670623
Patent Document 5 Japan Patent No. 5196848
Patent Document 6: Japan Patent No. 5522820
Patent Document 7: Japan Patent No. 5077984
Patent Document 8: JP-A-H10-234381

### Summary of the Invention

### Problems to be solved by the Invention

Under such circumstances, the inventors of the present invention have chosen, among soil-borne plant pathogens, microorganisms belonging to the genus Plasmodiophora which cause clubroot disease in Chinese cabbage and the like, microorganisms belonging to the genus Pythium which cause damping off disease and the like in Cucurbitaceous vegetables and the like, and microorganisms belonging to the genus Phytophthora which causes blight of potato and the like, as detection target microorganisms.

Then, the inventors of the present invention made intensive studies, and have successfully developed a primer set capable of preferably amplifying, as a target region, a β-tubulin gene in DNA of these detection target microorganisms and a primer set capable of preferably amplifying, as a target region, of an ITS region in rDNA gene (Ribosomal RNA gene) of these detection target microorganisms, and at the same time, have developed a microarray provided with a probe (a single chain oligo DNA on a DNA chip substrate) capable of identifying each amplification product, and succeeded in specifically detecting these pathogenic microorganisms.

Patent Document 1 discloses oligonucleotides for detecting or identifying microorganisms belonging to the genus Pythium simply, rapidly and with high sensitivity. Further, Patent Document 2 discloses a detection method and a primer for detecting pathogens causing a major disease of strawberry that are capable of detect microorganisms belonging to the-genus Phytophthora.

However, with these technologies, it was not possible to detect microorganisms-belonging to the genus Plasmodiophora. In addition, it is desired that microorganisms belonging to the genus Plasmodiophora, microorganisms belonging to the genus Pythium and microorganisms belonging to the genus Phytophthora be preferably detected specifically and simultaneously.

In the case where a DNA chip is used as an identification method using genes in fungi detection, first, a probe that specifically is coupled with a target region in DNA of a fungi as a detection target is prepared and immobilized on a DNA chip. Then, fungi collected from the environment, a product or a plant specimen is cultured, the fungi DNA is extracted, and the target region in the fungi DNA is amplified by a PCR method or the like. Further, the amplification product is dropped on a DNA chip, and the amplification product is hybridized with the probe, and the fungi is detected by measuring the fluorescence intensity etc. of a label contained in the amplification product.

Incidentally, since there are various kinds of fungi to be detected in the environment, it is desirable that a plurality of fungi can be simultaneously detected by using a single DNA chip. For this purpose, it is necessary to immobilize probes selected from a plurality of fungi on a DNA chip.

On the other hand, the amplification product of the target region to be hybridized with a probe can be obtained by a PCR method or the like using a predetermined primer set, but depending on the type of fungi, a single target region tends to cause a false positive reaction. Because of this, there is a case where it is desirable to simultaneously amplify plural target regions with plural primer sets. Further, instead of individually amplifying the target regions of cultured fungi, by simultaneously amplifying plural kinds of fungi and detecting fungi by a DNA chip on which probes selected from these plural kinds of fungi are immobilized, the speed of detection improves.

However, in the case of conducting multiplexing to simultaneously amplify plural target regions, a probe that functions with higher accuracy is required. In addition, if the number of fungi to be amplified simultaneously increases, the accuracy required for probes is further increased.

The inventors of the present invention selected four kinds of plant pathogens, i.e. Colletotrichum acutatum, Fusarium solani, Alternaria solani, and Rhizoctonia solani as a detection target fungi, and made extensive studies to prepare a carrier for detecting fungi capable of detecting these.

Among the specific target regions in DNA of each of these plural kinds of fungi, if a probe is prepared by selecting a base sequence that is specifically coupled with each fungi and that is not coupled with other kinds of fungi, it is theoretically possible to specifically detect these plural types of fungi by each probe.

Actually, however, probes obtained in this manner do not necessarily function effectively. Therefore, in order to prepare each probe, it was necessary to find a probe that effectively functions by repeating trial and error while confirming the effect of the probe by experiments.

Here, a primer for detecting Colletotrichum acutatum is described in Patent Document 6, a probe for detecting Fusarium solani is described in Patent Document 7, a primer and a probe for detecting Rhizoctonia solani are described in Patent Document 8.

However, a carrier for detecting fungi which is immobilized a probe capable of simultaneously and specifically detecting with high accuracy four types of fungi, i.e. Colletotrichum acutatum, Fusarium solani, Altanaria solani, and Rhizoctonia solani, has not been known.

The present invention has been attained in view of the above circumstances, and an object thereof is to provide a method for detecting microorganisms, a kit for detecting microorganisms, a microarray for detecting microorganisms capable preferably detecting microorganisms belonging to the genera Plasmodiophora, Pythium and Phytophthora.

Another object of the present invention is to provide a carrier for detecting fungi, a method for detecting fungi and a kit for detecting fungi capable of simultaneously detecting rapidly and specifically with a high degree of accuracy four types of fungi as plant pathogens, i.e. Colletotrichum acutatum, Fusarium solani, Altanaria solani and Rhizoctonia solani.

### Means for Solving the Problems

In order to attain the above-mentioned object, the method for detecting microorganism of the present invention is a method for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
wherein the PCR is conducted, for a β-tubulin gene in DNA of microorganisms belonging to the genus Plasmodiophora as the PCR amplification target, with the use of the extracted DNA and a first primer set which comprises a forward primer comprising at least one of base sequences represented by SEQ ID NOS: 1-3 and a reverse primer comprising at least one of base sequences represented by SEQ ID NOS: 4 and 5, and then the presence or absence of the microorganisms belonging to the genus Plasmodiophora in the sample is determined on the basis of the obtained amplification product.

Further, the kit for examining microorganism according to the present invention is a kit for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microorganisms test kit being characterized by comprising a primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for β-tubulin genes in DNA of microorganisms belonging to the genera Plasmodiophora, Pythium and Phytophthora.

Further, the microarray for detecting microorganism according to the present invention is a microarray for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microarray being characterized by immobilizing a first probe, which comprises at least one of a base sequence represented by SEQ ID NO: 12 and a complementary sequence of said base sequence represented by SEQ ID NO: 12, to be complementarily coupled with a first amplification product obtained by PCR with the use of a first primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for DNA of microorganisms belonging to the genus Plasmodiophora and a β-tubulin gene in DNA of microorganisms belonging to the genus Plasmodiophora.

The carrier for detecting fungi according to the present invention is a carrier for detecting fungi characterized by immobilizing two or more probes selected from probe groups having base sequences enumerated in the following (a), (b) or (c):
(a) probes having base sequences represented by SEQ ID NOS: 101 to 106,
(b) probes which are hybridizable under stringent conditions with nucleic acid fragments comprising complementary sequences to base sequences represented by SEQ ID NOS: 101 to 106, and
(c) probes having base sequences complementary to the probes of (a) or (b).

The carrier for detecting fungi according to the present invention is preferably one obtained by immobilizing a probe selected from each of the following groups of:
a first probe group comprising a probe, to detect Colletotrichum acutatum, having a base sequence represented by SEQ ID NO: 101 selected from an ITS region, and a probe having a base sequence represented by SEQ ID NO: 102 selected from β-tubulin genes,
a second probe group comprising a probe, to detect Fusarium solani, having a base sequence represented by SEQ ID NO: 103 selected from an ITS region, and a probe having a base sequence represented by SEQ ID NO: 104 selected from β-tubulin genes,
a third probe group comprising a probe, to detect Alternaria solani, having a base sequence represented by SEQ ID NO: 105 selected from an ITS region, and
a fourth probe group comprising a probe, to detect Rhizoctonia solani, having a base sequence represented by SEQ ID NO: 106 selected from an ITS region.

The method for detecting fungi according to the present invention is a method for detecting fungi which comprises a step amplifying a target region in DNA of the fungi as a detection target by PCR to confirm the presence or absence of an amplification product, the method for detecting fungi comprising:
including, in a sample, a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110,
in a case where at least one DNA of Colletotrichum acutatum, Fusarium solani, Alternaria solani and Rhizoctonia solani is contaned in the sample,
amplifying a target region in the DNA contained in the sample with the use of a reaction solution for the PCR, and
dropping an obtained amplification product on the carrier for fungi detection mentioned above to couple the obtained amplification product with a probe having a complementary base sequence.

The kit for detecting fungi according to the present invention is a kit for detecting fungi comprising a primer set to amplify a target region in DNA of fungi as a detection target, and a carrier immobilizing a probe to confirm the presence or absence of an amplification product, wherein:
the primer set comprises:
   a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110, and
   the carrier immobilizes:
      a probe selected from each of the groups of:
         a first probe group comprising a probe, to detect Colletotrichum acutatum, having a base sequence represented by SEQ ID NO: 101 selected from an ITS region, and a probe having a base sequence represented by SEQ ID NO: 102 selected from β-tubulin genes,
         a second probe group comprising a probe, to detect Fusarium solani, having a base sequence represented by SEQ ID NO: 103 selected from an ITS region, and a probe having a base sequence represented by SEQ ID NO: 104 selected from β-tubulin genes,
         a third probe group comprising a probe, to detect Alternaria solani, having a base sequence represented by SEQ ID NO: 105 selected from an ITS region, and
         a fourth probe group comprising a probe, to detect Rhizoctonia solani, having a base sequence represented by SEQ ID NO: 106 selected from an ITS region.

In the specification and the claims of the present invention, the "probe group" means an assembly of probes. The probe group comprises not only a plurality of probe but also a single probe.

### Advantageous Effects of the Invention

According to the present invention, it is possible to detect preferably microorganisms belonging to the genera Plasmodiophora, Pythium and Phytophthora.

Further, according to the present invention, it is possible to simultaneously detect rapidly and specifically with a high degree of accuracy plant pathogens, i.e. Colletotrichum acutatum, Fusarium solani, Altanaria solani, and Rhizoctonia solani.

### Brief Explanation of the Drawings

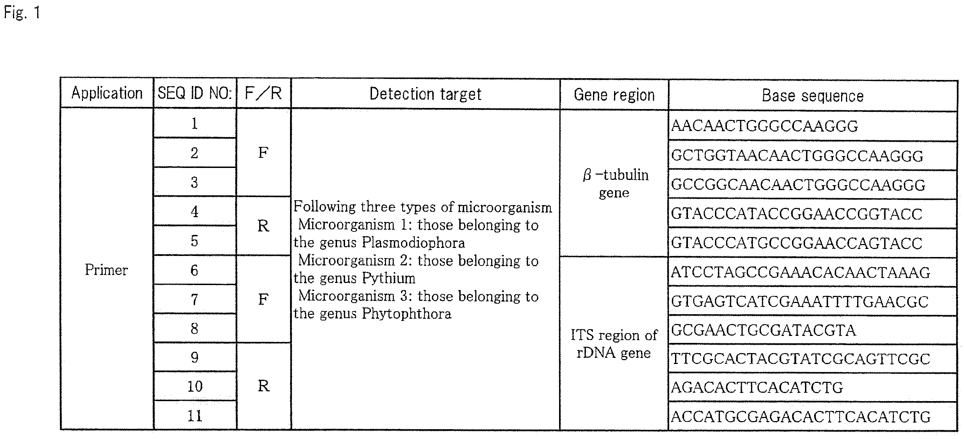

Fig. 1 is a view showing base sequences of primers used in the method for detecting microorganism, the kit for detecting microorganism and the microarray for detecting microorganism according to the embodiment of the present invention;

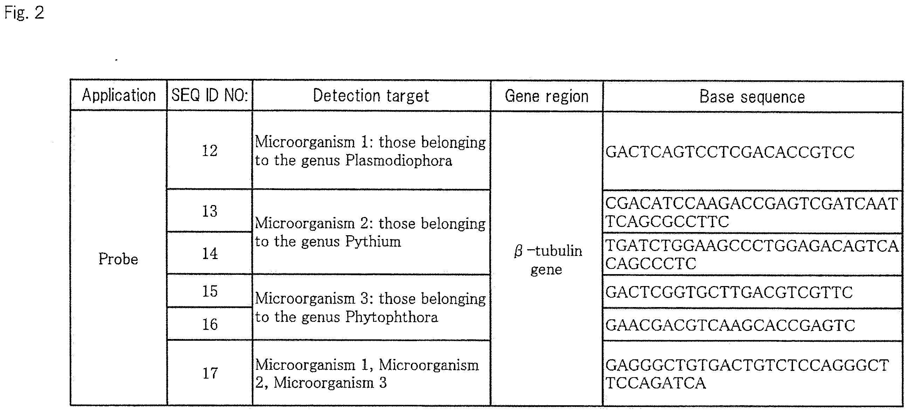

Fig. 2 is a view showing base sequences of probes targeting a β-tubulin gene used in the method for detecting microorganism, the kit for detecting microorganism and the microarray for detecting microorganism according to the embodiment of the present invention;

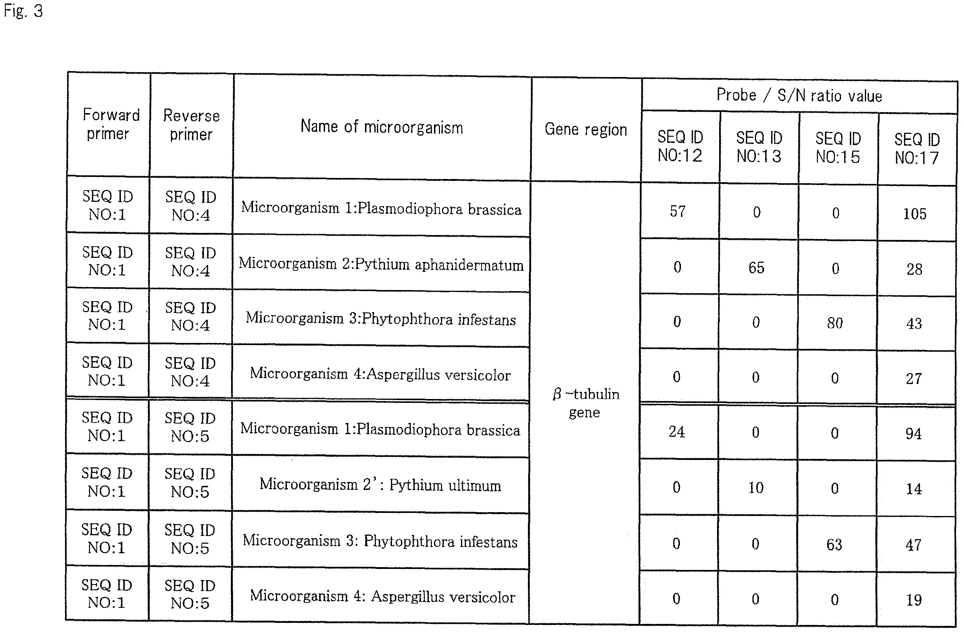

Fig. 3 is a view showing the results (test 1) of detecting microorganisms belonging to the genera Plasmodiophora, Pythium and Phytophthora by using the method for detecting microorganism, the kit for detecting microorganism and the microarray for detecting microorganism according to the embodiment of the present invention;

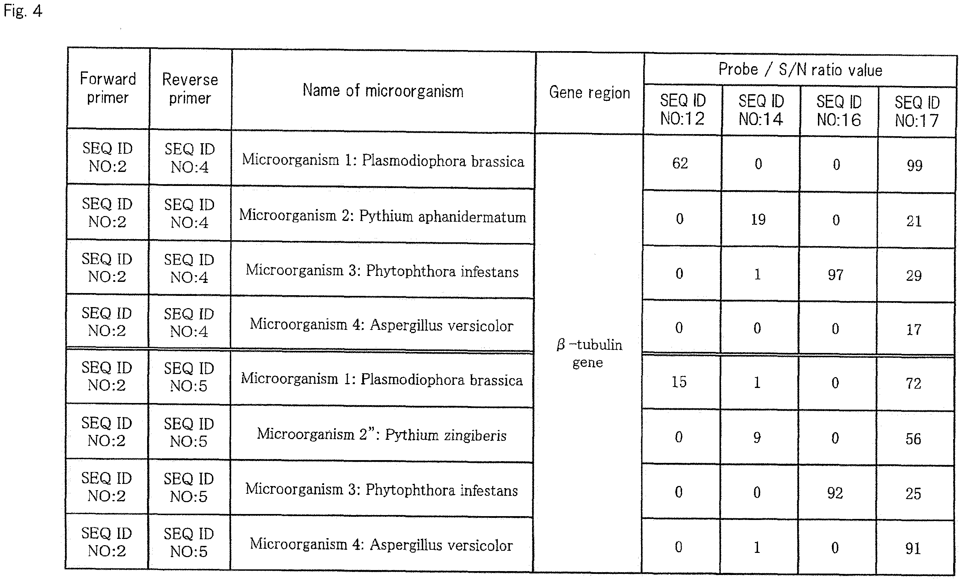

Fig. 4 is a view showing the results (test 2) of detecting microorganisms belonging to the genera Plasmodiophora, Pythium and Phytophthora by using the method for detecting microorganism, the kit for detecting microorganism and the microarray for detecting microorganism according to the embodiment of the present invention;

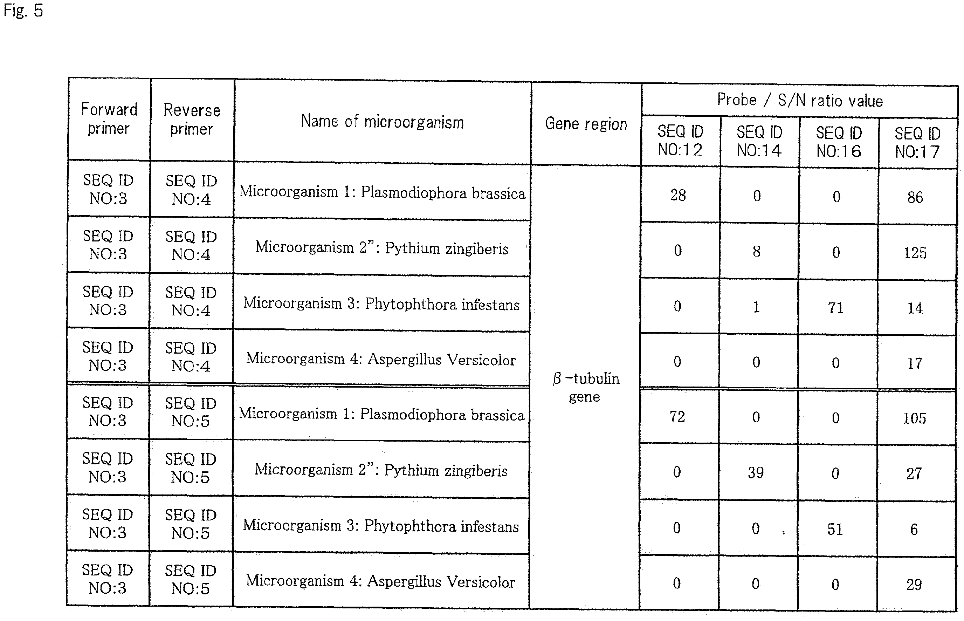

Fig. 5 is a view showing the results (test 3) of detecting microorganisms belonging to the genera Plasmodiophora, Pythium and Phytophthora by using the method for detecting microorganism, the kit for detecting microorganism and the microarray for detecting microorganism according to the embodiment of the present invention;

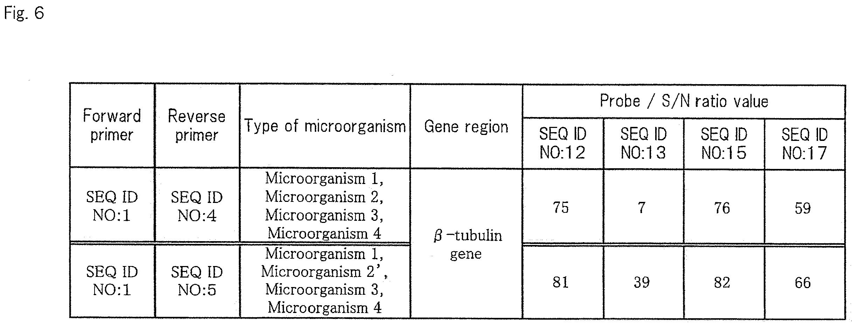

Fig. 6 is a view showing the results (test 4) of detecting microorganisms belonging to the genera Plasmodiophora, Pythium and Phytophthora by using the method for detecting microorganism, the kit for detecting microorganism and the microarray for detecting microorganism according to the embodiment of the present invention;

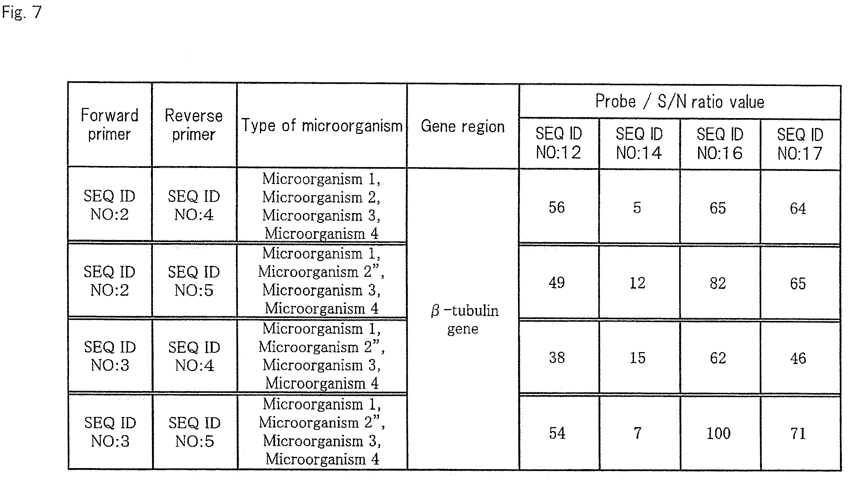

Fig. 7 is a view showing the results (test 5) of detecting microorganisms belonging to the genera Plasmodiophora, Pythium and Phytophthora by using the method for detecting microorganism, the kit for detecting microorganism and the microarray for detecting microorganism according to the embodiment of the present invention;

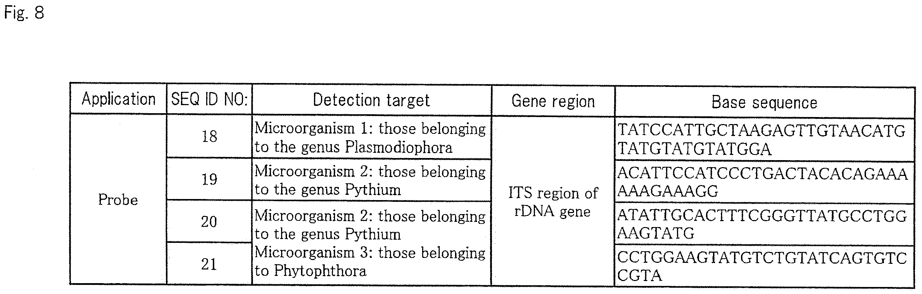

Fig. 8 is a view showing base sequences of probes targeting an ITS region in rDNA gene used in the method for detecting microorganism, the kit for detecting microorganism and the microarray for detecting microorganism according to the embodiment of the present invention;

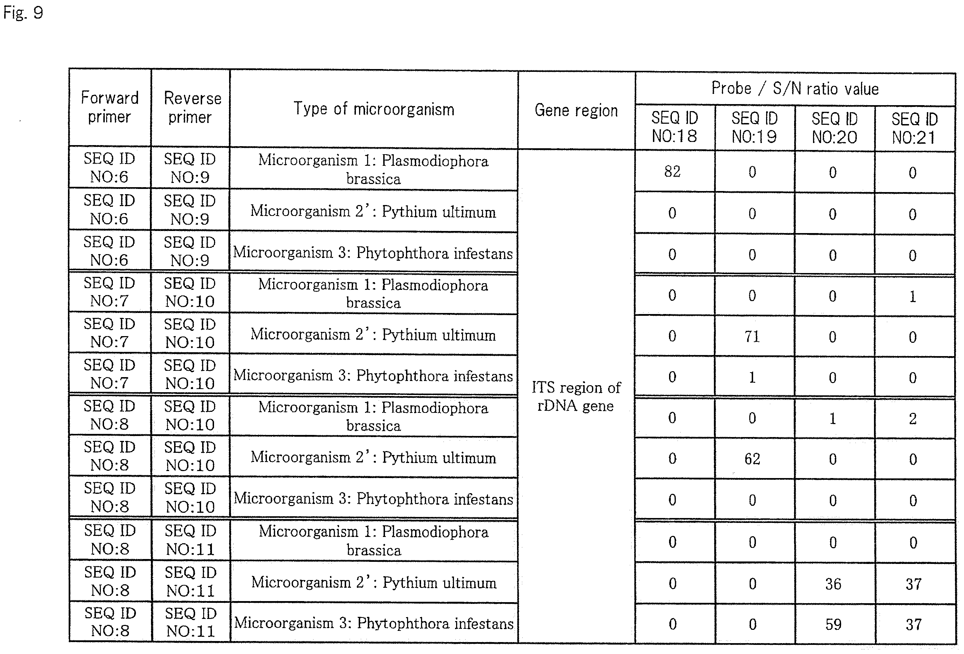

Fig. 9 is a view showing the results (test 6) of detecting microorganisms belonging to the genera Plasmodiophora, Pythium and Phytophthora by using the method for detecting microorganism, the kit for detecting microorganism and the microarray for detecting microorganism according to the embodiment of the present invention;

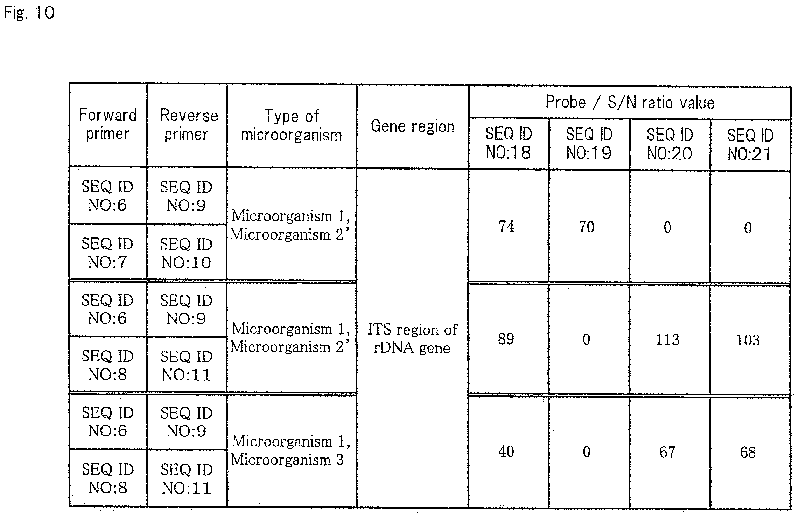

Fig. 10 is a view showing the results (test 7) of detecting microorganisms belonging to the genera Plasmodiophora, Pythium and Phytophthora by using the method for detecting microorganism, the kit for detecting microorganism and the microarray for detecting microorganism according to the embodiment of the present invention;

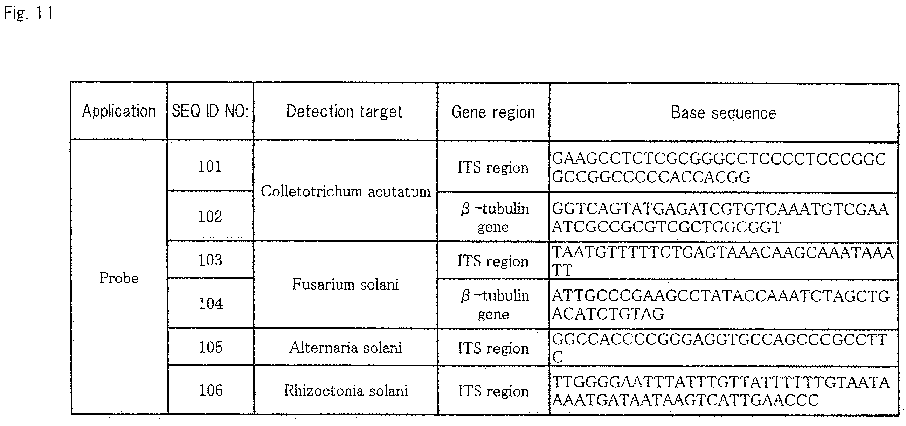

Fig. 11 is a view showing base sequences of probes to be immobilized on the carrier for detecting fungi according to the embodiment of the present invention;

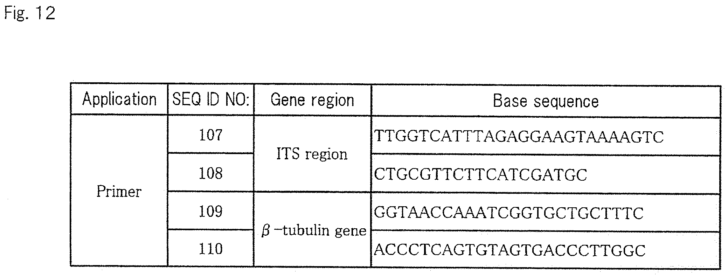

Fig. 12 is a view showing base sequences of primers for amplifying a target region of fungi to be detected by the carrier for detecting fungi according to the embodiment of the present invention; and

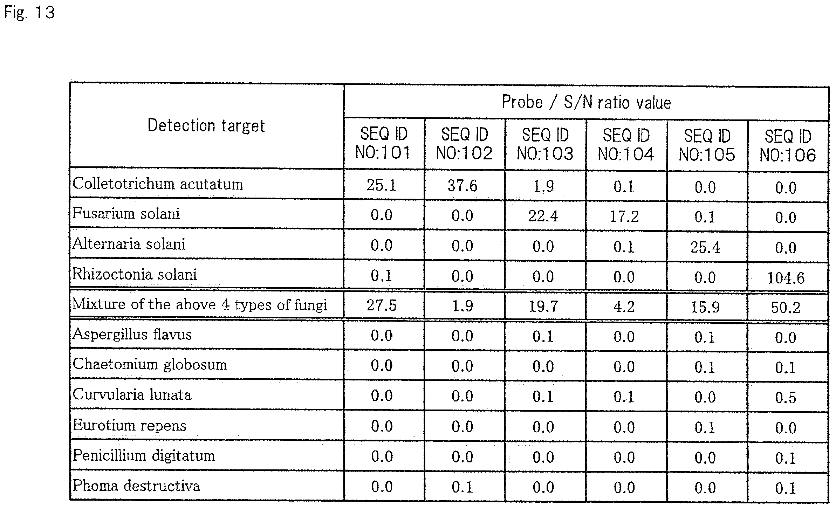

Fig. 13 is a view showing the fluorescent intensity (S/N ratio values) detected for probes immobilized on the carrier for detecting fungi according to the embodiment of the present invention.

### Mode for Carrying out the Invention

Hereinbelow, a detailed explanation is made on the method for detecting microorganism, the kit for detecting microorganism and the microarray for detecting microorganism according to an embodiment of the present invention.

The method for detecting microorganism according to the present embodiment is characterized in that it comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
wherein the PCR is conducted, for a β-tubulin gene in DNA of microorganisms belonging to the genus Plasmodiophora as the PCR amplification target, with the use of the extracted DNA and a first primer set which comprises a forward primer comprising at least one of base sequences represented by SEQ ID NOS: 1-3 and a reverse primer comprising at least one of base sequences represented by SEQ ID NOS: 4 and 5, and then the presence or absence of the microorganisms belonging to the genus Plasmodiophora in the sample is determined on the basis of the obtained amplification product.

The method for detecting microorganism according to the present embodiment is characterized in that a first amplification product obtained through the PCR with the use of DNA of microorganisms belonging to the genus Plasmodiophora and the first primer set is brought into contact with a microarray immobilizing a first probe, which comprises at least one of a base sequence represented by SEQ ID NO: 12 and a complementary sequence of said base sequence represented by SEQ ID NO: 12, to be complementarily coupled with the first amplification product, and
a label of the first amplification product complementarily coupled with the first probe is detected.

Microorganism belonging to the genus Plasmodiophora are soil-borne plant pathogens, which are parasitic on cruciferous vegetables such as Chinese cabbage, cabbage and the like, and cause clubroot disease. They are obligate parasites that infest only living plant cells to cause disease.

In this specification, all base sequences are to be grasped from the 5' terminal to the 3' terminal direction.

According to the method for detecting microorganism according to the present embodiment, by conducting PCR by using the first primer set mentioned above, a β-tubulin gene in DNA of microorganisms belonging to the gene Plasmodiophora can be preferably amplified.

Further, by using the microarray on which the first probe is immobilized mentioned above, microorganisms belonging to the gene Plasmodiophora can be specifically detected.

The method for detecting microorganism according to the present embodiment is a method in which the PCR is conducted, for a β-tubulin gene in DNA of microorganisms belonging to the genera Plasmodiophora, Pythium and Phytophthora as PCR amplification targets, with the use of the first primer set and the extracted DNA, and
the presence or absence of microorganisms belonging to the genera Plasmodiophora, Pythium and Phytophthora in the sample is simultaneously determined on the basis of the obtained amplification product.

Further, the method for detecting microorganism of the present embodiment is characterized in that a second amplification product obtained through the PCR with the use of DNA of microorganisms belonging to the genus Pythium and the first primer set is brought into contact with a microarray immobilizing a second probe, which comprises at least one of base sequences represented by SEQ ID NOS: 13 and 14 and complementary sequences of said base sequences represented by SEQ ID NOS: 13 and 14, to be complementarily coupled with the second amplification product, and
a label of the second amplification product complementarily coupled with the second probe is detected.

Microorganisms belonging to the genus Pythium are multi-offensive soil-borne plant pathogens, causing damping-off disease and soft rot pathogen, etc. on many kinds of vegetable such as cucurbitaceous vegetables and eggplant vegetables.

Further, the method for detecting microorganism according to the present embodiment is characterized in that a third amplification product obtained through the PCR with the use of DNA of microorganisms belonging to the genus Phytophthora and the first primer set is brought into contact with a microarray immobilizing a third probe, which comprises at least one of base sequences represented by SEQ ID NOS: 15 and 16, to be complementarily coupled with the third amplification product, and
a label of the third amplification product complementarily coupled with the third probe is detected.

Microorganism belonging to the genus Phytophthora are plant pathogens that live in soil and in plants, and Phytophthora infestans that caused potato famine in Europe in the mid-nineteenth century is famous.

According to the method for detecting microorganism according to the present embodiment, by conducting PCR by using the first primer set as mentioned above, it is possible to preferably amplify a β-tubulin gene in DNA of microorganisms belonging to the genera Pythium and Phytophthora.

Further, by using the microarray on which the second probe is immobilized as mentioned above, it is possible to detect microorganisms belonging to the genus Pythium specifically, and by using the microarray on which the third probe is immobilized as mentioned above, it is possible to specifically detect microorganisms belonging to the genus Phytophthora.

Further, according to the method for detecting microorganism according to the present embodiment, by conducting PCR by using the first primer set as mentioned above, it is possible to preferably amplify a β-tubulin gene in DNA of microorganisms belonging to the genera Plasmodiophora, Pythium and Phytophthora, and by using the microarray on which the first probe, the second probe and the third probe are immobilized, it is possible to detect specifically and simultaneously these microorganisms.

Further, the method for detecting microorganism according to the present embodiment is a method for detecting microorganism, wherein a fourth amplification product obtained through the PCR with the use of DNA of at least one of the microorganisms belonging to the genera Plasmodiophora, Pythium and Phytophthora and other microorganisms and the first primer set is brought into contact with a microarray immobilizing a fourth probe, which comprises at least one of a base sequence represented by SEQ ID NO: 17 and a complementary sequence of said base sequence represented by SEQ ID NO: 17, to be complementarily coupled with a fourth amplification product, and
a label of the fourth amplification product complementarily coupled with the fourth probe is detected.

According to the method for detecting microorganism according to the present embodiment, by using the microarray on which the fourth probe is immobilized as mentioned above, it is possible to detect microorganisms belonging to the genera Plasmodiophora, Pythium and Phytophthora, and other microorganisms such as Aspergillus versicolor.

Meanwhile, the other microorganisms are not limited to Aspergillus versicolor.- The microarray on which the fourth probe is immobilized as mentioned above may also be used in common for detecting various fungi.

The method for detecting microorganism according to the present embodiment is characterized in that the PCR is conducted, for an ITS region of rDNA gene in DNA of microorganisms belonging to the genus Plasmodiophora as the PCR amplification target, with the use of the extracted DNA and a second primer set which comprises a forward primer comprising a base sequence represented by SEQ ID NO: 6 and a reverse primer comprising a base sequence represented by SEQ ID NO: 9, and
the presence or absence of the microorganisms belonging to the genus Plasmodiophora in the sample is determined on the basis of the obtained amplification product.

Further, the method for detecting microorganism according to the present embodiment is characterized in that a fifth amplification product obtained through the PCR with the use of DNA of microorganisms belonging to the genus Plasmodiophora and the second primer set is brought into contact with a microarray immobilizing a fifth probe, which comprises at least one of a base sequence represented by SEQ ID NO: 18 and a complementary sequence of the base sequence represented by SEQ ID NO: 18, to be complementarily coupled with the fifth amplification product, and
a label of the fifth amplification product complementarily coupled with the fifth probe is detected.

According to the method for detecting microorganism according to the present embodiment, by conducting PCR using the second primer set as mentioned above, it is possible to preferably amplify an ITS region of rDNA gene in DNA of microorganisms belonging to the genus Plasmodiophora.

Further, by using the microarray on which the fifth probe as mentioned above is immobilized, it is possible to specifically detect microorganisms belonging to the genus Plasmodiophora.

Further, the method for detecting microorganism according to the present embodiment is characterized in that the PCR is conducted, for an ITS region of rDNA gene in DNA of microorganisms belonging to the genus Pythium as the PCR amplification target with the use of the extracted DNA and a third primer set which comprises a forward primer comprising at least one of base sequences represented by SEQ ID NOS: 7 and 8 and a reverse primer comprising a base sequence represented by SEQ ID NO: 10, and
the presence or absence of the microorganisms belonging to the genus Pythium in the sample is determined on the basis of the obtained amplification product.

The method for detecting microorganism according to the present embodiment is characterized in that a sixth amplification product obtained through the PCR with the use of DNA of microorganisms belonging to the genus Pythium and the third primer set is brought into contact with a microarray immobilizing a sixth probe, which comprises at least one of a base sequence represented by SEQ ID NO: 19 and a complementary sequence of said base sequence represented by SEQ ID NO: 19, to be complementarily coupled with the sixth amplification product, and
a label of the sixth amplification product complementarily coupled with the sixth probe is detected.

According to the method for detecting microorganism according to the present embodiment, by conducting PCR using the third primer set as mentioned above, it is possible to preferably amplify an ITS region of rDNA gene in DNA of microorganisms belonging to the genus Pythium.

Further, by using the microarray on which the sixth probe as mentioned above is immobilized, it is possible to detect specifically microorganisms belonging to the genus Pythium.

According to the method for detecting microorganism according to the present embodiment, by conducting PCR using the second primer set and the third primer set, it is possible to preferably amplify ITS regions of rDNA gene in DNA of microorganisms belonging to the genera Plasmodiophora and Pythium, and by using the microarray on which the fifth probe and the sixth probe are immobilized, it is possible to detect microorganisms belonging to the genera Plasmodiophora and Pythium specifically and simultaneously.

Further, the method for detecting microorganism according to the present embodiment is a method characterized in that the PCR is conducted, for an ITS region of rDNA gene in DNA of microorganisms belonging to the genus Pythium or Phytophthora as the PCR amplification target, with the use of the extracted DNA and a fourth primer set which comprises a forward primer comprising a base sequence represented by SEQ ID NO: 8 and a reverse primer comprising a base sequence represented by SEQ ID NO: 11, and
the presence or absence of the microorganisms belonging to the genus Pythium or Phytophthora in the sample is determined on the basis of the obtained amplification product.

The method for detecting microorganism according to the present embodiment is characterized in that a seventh amplification product obtained through the PCR with the use of DNA of microorganisms belonging to the genus Pythium or Phytophthora and the fourth primer set is brought into contact with a microarray immobilizing a seventh probe, which comprises at least one of base sequences represented by SEQ ID NOS: 20 and 21 and complementary sequences of at least one of the base sequences represented by SEQ ID NOS: 20 and 21, to be complementarily coupled with the seventh amplification product, and
a label of the seventh amplification product complementarily coupled with the seventh probe is detected.

According to the method for detecting microorganism according to the present embodiment, by conducting PCR using the fourth primer set as mentioned above, it is possible to preferably amplify ITS regions of rDNA gene in DNA of microorganisms belonging to the genera Pythium and Phytophthora.

Further, by using the microarray on which the seventh probe is immobilized, it is possible to detect microorganisms belonging to the genera Pythium and Phytophthora.

In the method for detecting microorganism according to the present embodiment, when a primer set targeting an ITS region of rDNA gene is used as a primer set without using the first primer set targeting a β-tubulin gene, by using in combination the third primer set (SEQ ID NOS: 7, 8, 10) and the fourth primer set (SEQ ID NOS: 8 and 11), and by using the microarray on which the sixth probe (SEQ ID NO: 19 + complementary sequence) and the seventh probe (SEQ ID NOS: 20, 21 + complementary sequence), the following judgment can be conducted for the detection of microorganisms belonging to the genera Pythium and Phytophthora.

First, if a label of an amplification product is detected for both the sixth probe and the seventh probe, it is understood that microorganisms belonging to the genus Pythium are present and the presence or absence of microorganisms belonging to the genus Phytophthora is unclear. Further, a label of an amplification product is not detected for the sixth probe and a label of an amplification product is detected for the seventh probe, it is understood that microorganisms belonging to the genus Pythium are not present, and microorganisms belonging to the genus Phytophthora are present. Further, if no label of an amplification product is detected for both the sixth probe and the seventh probe, it is understood that neither of microorganisms belonging to the genus Pythium and microorganisms belonging to the genus Phytophthora is present.

In the method for detecting microorganism according to the present embodiment, as mentioned above, when a first primer set targeting a β-tubulin gene is used as the primer set, by using the microarray on which a first probe (SEQ ID NO. 12 + complementary sequence), a second probe (SEQ ID NOS: 13 and 14 + complementary sequence) and a third probe (SEQ ID NOS: 15 and 16), it is possible to detect specifically and simultaneously microorganisms belonging to the genera Plasmodiophora, Pythium and Phytophthora.

The kit for detecting microorganism according to the present embodiment is characterized in that it comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microorganisms test kit being characterized by comprising a primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for a β-tubulin gene in DNA of microorganisms belonging to the genera Plasmodiophora, Pythium and Phytophthora.

According to the kit for detecting microorganism according to the present embodiment, by conducting PCR using such primer set, it is possible to amplify preferably β-tubulin genes in DNA of microorganisms belonging to the genera Plasmodiophora, Pythium and Phytophthora.

The kit for detecting microorganism according to the present embodiment is characterized in that it includes a primer set which comprises a forward primer comprising a base sequence represented by SEQ ID NO: 6 and a reverse primer comprising a base sequence represented by SEQ ID NO: 9 as targets of amplification by the PCR for an ITS region of a rDNA gene in DNA of microorganisms belonging to the genus Plasmodiophora.

According to the kit for detecting microorganism according to the present embodiment, by conducting PCR using such primer set, it is possible to preferably amplify an ITS region of rDNA gene in DNA of microorganisms belonging to the genus Plasmodiophora.

Further, according to the kit for detecting microorganism according to the present embodiment, by conducting PCR using each of the above-mentioned primer sets, it is possible to preferably amplify simultaneously β-tubulin genes in DNA of microorganisms belonging to the genera Plasmodiophora, Pythium and Phytophthora, and an ITS region of rDNA gene in DNA of microorganisms belonging to the genus Plasmodiophora.

Further, the kit for detecting microorganism according to the present embodiment is characterized in that it includes a primer set which comprises a forward primer comprising any one of base sequences represented by SEQ ID NOS: 7 and 8 and a reverse primer comprising a base sequence represented by SEQ ID NO: 10 as targets of amplification by the PCR for an ITS region of rDNA gene in DNA of microorganisms belonging to the genus Pythium.

According to the kit for detecting microorganism according to the present embodiment, by conducting PCR using such primer set, it is possible to preferably amplify an ITS region of rDNA gene in DNA of microorganisms belonging to the genus Pythium.

Further, according to the kit for detecting microorganism according to the present embodiment, by conducting PCR using each of the above-mentioned primer kits, it is possible to preferably amplify simultaneously β-tubulin genes in DNA of microorganisms belonging to the genera Plasmodiophora, Pythium and Phytophthora, and ITS regions of rDNA gene in DNA of microorganisms belonging to the genera Plasmodiophora and Pythium.

Further, the kit for detecting microorganism according to the present embodiment is characterized in that it further includes a primer set which comprises a forward primer comprising a base sequence represented by SEQ ID NO: 8 and a reverse primer comprising a base sequence represented by SEQ ID NO: 11 as targets of amplification by the PCR for an ITS region of rDNA gene in DNA of microorganisms belonging to the genus Pythium or Phytophthora.

According to the kit for detecting microorganism according to the present embodiment, by conducting PCR using such primer set, it is possible to preferably amplify ITS regions of rDNA gene in DNA of microorganisms belonging to the genera Pythium and Phytophthora.

Further, according to the kit for detecting microorganism according to the present embodiment, by conducting PCR using each of the above-mentioned primer sets, it is possible to preferably and simultaneously amplify β-tubulin genes in DNA of microorganisms belonging to the genera Plasmodiophora, Pythium and Phytophthora, and ITS regions of rDNA gene in DNA of microorganisms belonging to the genera Plasmodiophora, Pythium and Phytophthora.

A method of extracting DNA from a sample when using the method for detecting microorganism and a kit for detecting microorganism according to the present embodiment is not particularly limited, but can be conducted as follows, for example.

First, colonies of microorganisms are collected, colonies are frozen with liquid nitrogen or the like, and the cells of the microorganisms contained in the colonies are crushed. Next, genomic DNA is extracted from the obtained crushed cells of the microorganisms. Extraction of genomic DNA can be performed by a common method such as a method based on the CTAB method (Cetyl trimethyl ammonium bromide) or a method using a DNA extraction apparatus. It is also possible to extract fungal DNA directly from the soil. Also in this case, extraction of DNA can be performed by a common method such as a direct lysis method or a method using a soil DNA extraction kit.

Next, the target region in the extracted genomic DNA is amplified. That is, a DNA fragment containing a target region in genomic DNA is amplified. As a method for amplifying the target region, a PCR method can be preferably used. As a PCR apparatus, a common thermal cycler or the like can be used.

As a reaction solution for PCR, for example, it is preferable to use one having the following composition. It is preferable to use a PCR reaction solution containing a nucleic acid synthesis substrate (dNTPmixture (dCTP, dATP, dTTP, dGTP)), a primer set, a nucleic acid synthesis enzyme (EX Taq HotStart DNA polymerase, etc), a fluorescent labeling reagent (Cy5-dCTP, etc.), DNA of a sample, a buffer solution and water as a remaining component. As the buffer solution, Ampdirect (R) (manufactured by Shimadzu Corporation) can be used.

When using the method for detecting microorganism and the kit for detecting microorganism according to the present embodiment, the target region of each microorganism can be preferably amplified by conducting PCR under the following reaction conditions, for example.
(a) 95°C 10 min, (b) 95°C (DNA denaturation step) 30 sec, (c) 56°C (annealing step) 30 sec, (d) 72°C (DNA synthesis step) 60 sec ((b) to (d) are repeated 40 cycles), (e) 72°C 10 minutes

In the present embodiment and examples, primers and probes are not limited to the respective base sequences themselves. Those primers and probes in which one or several bases are deleted, substituted or added in each base sequence can be used. It is also possible to use nucleic acid fragments which is capable of hybridizing with nucleic acid fragments comprising a base sequence complementary to each base sequence under stringent conditions.

The stringent condition means a condition under which a specific hybrid is formed and a nonspecific hybrid is not formed. For example, the stringent condition means a condition under which a DNA having high homology 90% or more, preferably 95% or more of homology) to a DNA comprising each base sequence is hybridized with a DNA comprising a base sequence complementary to the DNA comprising each base sequence. Usually, the stringent condition is referred to as a case where hybridization occurs at a temperature that is lower by about 5°C to 30°C, preferably about 10°C to about 25°C, than the melting temperature (Tm) of a complete hybrid. As for the stringent condition, condition or the like described in J. Sambrook, et.al.: Molecular Cloning, A Laboratory Manual, Second Edition, Cold Spring Harbor Laboratory Press (1989), in particular, section 11.45, "Conditions for Hybridization of Oligonucleotide Probes" can be used.

A primer and a probe used in the method for detecting microorganism, the kit for detecting microorganism and the microarray for detecting microorganism each has a length of about 16 to 40 mer (base), and can be synthesized by a DNA synthesis apparatus.

The method for detecting microorganism according to the present embodiment is characterized in that it comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microarray being characterized by immobilizing a first probe, which comprises at least one of a base sequence represented by SEQ ID NO: 12 and a complementary sequence of said base sequence represented by SEQ ID NO: 12, to be complementarily coupled with a first amplification product obtained by PCR with the use of a first primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for DNA of microorganisms belonging to the genus Plasmodiophora and a β-tubulin gene in DNA of microorganisms belonging to the genus Plasmodiophora.

According to the microarray for detecting microorganism according to the present embodiment, by using the microarray on which the above-mentioned first probe is immobilized, it is possible to specifically detect microorganisms belonging to the genus Plasmodiophora.

The microarray for detecting microorganism according to the present embodiment is characterized by further immobilizing a second probe, which comprises at least one of base sequences represented by SEQ ID NOS: 13 and 14 and complementary sequences of said base sequences represented by SEQ ID NOS: 13 and 14, to be complementarily coupled with a second amplification product obtained through the PCR with the use of DNA of microorganisms belonging to the genus Pythium and the first primer set.

According to the microarray for detecting microorganism according to the present embodiment, by using the microarray on which the above-mentioned second probe is immobilized, it is possible to specifically detect the microorganisms belonging to the genus Pythium.

According to the microarray for detecting microorganism according to the present embodiment, by using the microarray on which the first probe and the second probe are immobilized, it is possible to specifically and simultaneously detect microorganisms belonging to the genera Plasmodiophora and Phytophthora.

The microarray for detecting microorganism according to the present embodiment is characterized by further immobilizing a third probe, which comprises at least one of base sequences represented by SEQ ID NOS: 15 and 16, to be complementarily coupled with a third amplification product obtained through the PCR with the use of DNA of microorganisms belonging to the genus Phytophthora and the first primer set.

According to the microarray for detecting microorganism according to the present embodiment, by using the microarray on which the above-mentioned third probe is immobilized, it is possible to specifically detect microorganisms belonging to the genus Phytophthora.

Further, according to the microarray for detecting microorganism according to the present embodiment, by using the microarray on which the first probe, the second probe and the third probe are immobilized, it is possible to specifically and simultaneously detect the genera Plasmodiophora, Pythium and Phytophthora.

Further, the microarray for detecting microorganism according to the present embodiment is characterized by further immobilizing a fourth probe, which comprises at least one of a base sequence represented by SEQ ID NO: 17 and a complementary sequence of said base sequence represented by SEQ ID NO: 17, to be complementarily coupled with a fourth amplification product obtained through the PCR with the use of DNA of at least one of microorganisms belonging to the genera Plasmodiophora, Pythium, Phytophthora and other microorganisms and the first primer set.

According to the microarray for detecting microorganism according to the present embodiment, by using the microarray on which the above-mentioned fourth probe is immobilized, it is possible to detect microorganisms belonging to the genera Plasmodiophora, Pythium and Phytophthora and other microorganisms such as Aspergillus versicolor.

Still further, the microarray for detecting microorganism according to the present embodiment is characterized by further immobilizing a fifth probe, which comprises at least one of a base sequence represented by SEQ ID NO: 18 and complementary sequence of said base sequence represented by SEQ ID NO: 18, to be complementarily coupled with a fifth amplification product obtained by the PCR with the use of a second primer set which comprises a forward primer comprising a base sequence represented by SEQ ID NO: 6 and a reverse primer comprising a base sequence represented by SEQ ID NO: 9 as targets of amplification by the PCR for DNA of microorganisms belonging to the genus Plasmodiophora and an ITS region of rDNA gene in DNA of microorganisms belonging to the genus Plasmodiophora.

According to the microarray for detecting microorganism according to the present embodiment, by using the microarray on which the above-mentioned fifth probe is immobilized, it is possible to specifically detect microorganisms belonging to the genus Plasmodiophora.

The microarray for detecting microorganism according to the present embodiment is characterized by further immobilizing a sixth probe, which comprises at least one of a base sequence represented by SEQ ID NO: 19 and a complementary sequence of said base sequence represented by SEQ ID NO: 19, to be complementarily coupled with a sixth amplification product obtained by the PCR with the use of a third primer set which consists of a forward primer comprising at least one of base sequences represented by SEQ ID NOS: 7 and 8 and a reverse primer comprising a base sequence represented by SEQ ID NO: 10 as targets of amplification by the PCR for DNA of microorganisms belonging to the genus Pythium and an ITS region of rDNA gene in DNA of microorganisms belonging to the genus Pythium.

According to the microarray for detecting microorganism according to the present embodiment, by using the above-mentioned probe on which the sixth probe is immobilized, it is possible to specifically detect microorganisms belonging to the genus Pythium.

Further, according to the microarray for detecting microorganism according to the present embodiment, by using the microarray on which the fifth probe and the sixth probe are immobilized, it is possible to specifically and simultaneously detect microorganisms belonging to the genera Plasmodiophora and Pythium.

Further, the microarray for detecting microorganism according to the present embodiment is characterized by further immobilizing a seventh probe, which comprises at least one of base sequences represented by SEQ ID NOS: 20 and 21 and complementary sequences of said base sequences represented by SEQ ID NOS: 20 and 21, to be complementarily coupled with a seventh amplification product obtained by the PCR with the use of a fourth primer set which comprises a forward primer comprising a base sequence represented by SEQ ID NO: 8 and a reverse primer comprising a base sequence represented by SEQ ID NO: 11 as targets of amplification by the PCR for DNA of microorganisms belonging to the genus Pythium or Phytophthora and an ITS region of rDNA gene in DNA of microorganisms belonging to the genus Pythium or Phytophthora.

According to the microarray for detecting microorganism according to the present embodiment, by using the above-mentioned probe on which the seventh probe is immobilized, it is possible to detect microorganisms belonging to the genera Pythium and Phytophthora.

The microarray for detecting microorganism according to the present embodiment is not particularly restricted as long as at least one of the above-mentioned probes for detecting microorganism as detection target is immobilized. For example, a spotted DNA microarray, a synthesized DNA microarray or the like can be used.

For example, when preparing an attached DNA chip as the microarray for detecting microorganism according to the present embodiment, this DNA chip can be prepared by immobilizing a probe on a glass substrate with a DNA spotter to form a spot corresponding to each probe. Further, when preparing a synthesized DNA chip, this DNA chip can be prepared by synthesizing a single-stranded oligo DNA having the above sequence on a glass substrate by optical lithography technology. The substrate is not limited to glass, and a plastic substrate, a silicon wafer, or the like can also be used. Further, the shape of the substrate is not limited to a plate-like shape, it may have various three-dimensional shapes. One into which a functional group is introduced on the surface so that a chemical reaction is possible can also be used.

Next, the amplification product is dropped on the microarray for detecting microorganism according to the present embodiment, and the presence or absence of the amplification product is confirmed by detecting a label of the amplification product hybridized with the probe immobilized on the microarray. As a result, it is possible to identify the microorganism as the detection target.

Detection of the label can be carried out using a common label detection device such as a fluorescence scanning device or the like. For example, detection can be conducted by measuring the fluorescence intensity of the amplification product using BIOSHOT (R) of Toyo Kohan Co., Ltd. It is preferable to obtain the measurement results as S/N ratio (Signal to Noise ratio) value. The S/N ratio value is calculated by (median fluorescence intensity value - background value) ÷ background value. The label is not limited to fluorescence, and others can be used.

As explained above, according to the method for detecting microorganism, the kit for detecting microorganism and the microarray for detecting microorganism according to the present embodiment, it is possible to preferably detect microorganisms belonging to the genera Plasmodiophora, Pythium, Phytophthora.

Hereinbelow, one embodiment of the carrier for detecting fungi, the method for detecting fungi and the kit for detecting fungi according to the present invention will be described in detail. The present invention is not limited to the specific contents of the present embodiment mentioned below and the Examples mentioned later.

### [Carrier for detecting fungi]

The carrier for detecting fungi according to the present embodiment is not particularly restricted as long as it is one on which at least one of probes (SEQ ID NOS: 101 to 106) to detect specific fungi shown in Fig. 11 is immobilized on a substrate. This carrier for detecting fungi can be produced as a spotted or synthesized DNA chip or a DNA microarray.

### (Detection target fungi)

The target fungi to be detected by the carrier for detecting fungi according to the present embodiment is four types; i.e. Colletotrichum acutatum, Fusarium solani, Alternaria solani and Rhizoctonia solani.

By confirming whether these types of fungi are present in soil or the like by using the carrier for detecting fungi according to the present embodiment, in planting plants, it is possible to prevent plant diseases caused by these pathogenic fungi. In addition, by confirming the absence of these types of fungi in soil, it is also possible to prevent application of excessive pesticide.

### (Probe)

The probe for detecting the target fungi according to the present embodiment is nucleic acid fragments comprising base sequences represented by SEQ ID NOS: 101 to 106. In the specification of the present invention, the SEQ ID NOS: 22-100 are missing.

Among these probes, one having a base sequence represented by SEQ ID NO. 101 is a probe selected from an ITS region for detecting Colletotrichum acutatum, and one having a base sequence represented by SEQ ID NO. 102 is a probe selected from a β-tubulin gene for detecting this fungus.

One having a base sequence represented by SEQ ID NO. 103 is a probe selected from an ITS region for detecting Fusarium solani. Further, one having a base sequence represented by SEQ ID NO. 104 is a probe selected from a β-tubulin gene for detecting this fungus.

One having a base sequence represented by SEQ ID NO. 105 is a probe selected from an ITS region for detecting Alternaria solani. Further, one having a base sequence represented by SEQ ID NO. 106 is a probe selected from an ITS region for detecting Rhizoctonia solani.

In the carrier for detecting fungi in the present embodiment, as mentioned above, as a probe, one selected from an ITS region and a β-tubulin gene in genomic DNA of fungi is used.

The ITS region is a portion spliced after transcription into RNA. Therefore, as compared with the coding region, it has poor preservability and is full of variety, but the similarity between the types of fungi is high and a false positive reaction occurs relatively often. Therefore, when immobilizing a large number of probes for fungi on the DNA chip and used for identification of fungi, depending on the type of fungi, if only the probe selected from the ITS region is used, the detection accuracy may be lowered. On the other hand, in the β-tubulin gene, there are relatively many unique sequences for each type of fungi. Therefore, in the carrier for detecting fungi of the present embodiment, depending on the type of fungi, it is possible to reduce occurrence of false positive reactions by using both the ITS region and the β-tubulin gene as the target region.

Specifically, with respect to Fusarium solani, by using both of a probe selected from an ITS region and a probe selected form a β-tubulin gene, it is better to judge that the fungus is detected only when a positive reaction is obtained for the both probes. Regarding this fungus, by doing this, it is possible to reduce a risk of false judgement based on occurrence of false positive reaction.

On the other hand, as for Colletotrichum acutatum, it can be judged that the fungus is present when a positive reaction is obtained for at least any one of a probe selected from an ITS region or a probe selected from a β-tubulin gene.

As for Alternaria solani and Rhizoctonia solani, when a positive reaction is obtained in a probe selected only from an ITS region, it can be judged that the fungi are present. The probe selected from the ITS region of these types of fungi is highly specific, and false positive reactions hardly occur relatively. Therefore, for these types of fungi, it is possible to detect by using a probe selected only from an ITS region.

Therefore, in the carrier for detecting fungi according to the present embodiment, as for Fusarium solani, it is preferred to judge to be present the fungus as the detection target when both a probe selected from an ITS region and a probe selected from a β-tubulin gene are immobilized and the both probes show a positive reaction. As for Colletotrichum acutatum, it is preferred to judge to be present the fungus as the detection target when both a probe selected from an ITS region and a probe selected from a β-tubulin gene are immobilized and one of these probes shows a positive reaction. As for the Alternaria solani and Rhizoctonia solani, it is preferred to judge to be present the fungi as the detection target when a probe selected only from an ITS region is immobilized and the probe shows a positive reaction.

### (Synthesis of probe)

Each of the above-mentioned probes immobilized on the carrier for detecting fungi of the present embodiment has a length of about 30 to 55 bases and can be synthesized by a common DNA synthesizer. As for all probes comprising base sequences represented by SEQ ID NOS: 101 to 106 immobilized on a carrier for detecting fungi in the Examples described later, those synthesized by a DNA synthesizer were used. Each of the primers used in PCR given in the Examples mentioned later that comprises base sequences represented by SEQ ID NOS: 107 to 110 has a length of about 19 to 26 bases and one synthesized by a DNA synthesizer was used.

The probes having base sequences represented by SEQ ID NOS: 101 to 106 in the present embodiment is not limited to one having said base sequences, and probes obtained by deletion, substitution or addition of one or several bases in the base sequences represented by SEQ ID NOS: 101 to 106 can be used. Probes capable of hybridizing under stringent conditions to nucleic acid fragments comprising base sequences complementary to the base sequences represented by SEQ ID NOS: 101 to 106 can also be used. Also, probes having base sequences complementary to the base sequences of these probes and probes comprising the base sequences represented by SEQ ID NOS: 101 to 106 can be used.

Also with respect to each primer comprising the base sequences of SEQ ID NO: 107 to 110 shown in Fig. 12 (the primer set of SEQ ID NOS: 107 and 108 and each primer in the primer set of SEQ ID NOS: 109 and 110), the primer is not limited to one comprising said sequence, and a primer obtained by deletion, substitution or addition of one or several bases in the base sequences represented by SEQ ID NOS: 107 to 110 and is capable of amplifying the same region as that amplified by the primer comprising the base sequences represented by the SEQ ID NOS: 107 to 110 can be used. Further, it is also possible to use a primer capable of hybridizing under stringent conditions to nucleic acid fragments comprising the base sequences complementary to the base sequences represented by SEQ ID NOS: 107 to 110 and is capable of amplifying the same region as that when the primer comprising the base sequences represented by the SEQ ID NOS: 107 to 110 can be used.

### (Preparation of carrier for detecting fungi)

The carrier for detecting fungi according to the present embodiment can be produced by an existing common method using probes having base sequences represented by the SEQ ID NOS: 101 to 106.

For example, when preparing an attached DNA chip as the carrier for detecting fungi according to the present embodiment, this DNA chip can be prepared by immobilizing on a glass substrate a probe by using a DNA spotter to form a spot corresponding to each probe. In addition, when preparing a synthesized DNA chip, this DNA chip can be prepared by synthesizing a single-stranded oligo DNA having the above sequence on a glass substrate by an optical lithography technique. The substrate is not limited to glass, and a plastic substrate, a silicon wafer, or the like can also be used. Further, the shape of the substrate is not limited to a plate-like shape, the substrate may have various three-dimensional shapes. One into which a functional group is introduced on the surface so that a chemical reaction is possible can also be used.

The carrier for detecting fungi according to the present embodiment may be one in which all or part of probes having base sequences represented by SEQ ID NOS: 101 to 106 is immobilized.

Further, the carrier for detecting fungi according to the present embodiment may be one in which at least one probe for detecting each type of fungus is immobilized.

Further, the carrier for detecting fungi according to the present embodiment may be one in which all or part of probes obtained by deletion, substitution or addition of one or several bases in the base sequences represented by SEQ ID NOS: 101 to 106 is immobilized or may be one in which all or part of probes capable of hybridizing under stringent conditions to nucleic acid fragments comprising base sequences complementary to the base sequences shown in SEQ ID NOS: 101 to 106. Also, it may be one in which all or part of probes having base sequences complementary to the base sequences of these probes or probes comprising base sequences represented by SEQ ID NOS: 101 to 106 is immobilized.

### [Method for detecting fungi]

The method for detecting fungi according to the present embodiment may be a method for detecting fungi which comprises a step amplifying a target region in DNA of the fungi of a detection target by PCR to confirm the presence or absence of an amplification product, the method for detecting fungi comprising:
including, in a sample, a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110,
in a case where at least one DNA of Colletotrichum acutatum, Fusarium solani, Alternaria solani and Rhizoctonia solani is contained in the sample,
amplifying a target region in the DNA contained in the sample with the use of a reaction solution for the PCR, and
dropping an obtained amplification product on the carrier for fungi detection to couple the obtained amplification product with a probe having a complementary base sequence. The method is not restricted to the configuration of the specific embodiment and the Examples. However, it comprises (1) collecting of fungi and extraction of DNA, (2) amplification of target region and (3) confirmation of amplification product.

### (1) Collection of fungi and extraction of DNA

For collecting fungi and extracting DNA, various methods can be used without particular restrictions. For example, a method can be mentioned in which soil for raising plants is collected, suspended in an appropriate amount of water, then diluted stage by stage at an arbitrary magnification, a diluted liquid at each stage is applied to the medium and cultured, and then DNA is extracted from the cultured fungi.

Alternatively, soil may be collected and DNA of soil microorganisms containing plant pathogenic fungi (microorganisms contains such as bacteria and nematodes) may be directly extracted from the collected soil. As the method therefor, a common method such as a direct lysis method or the use of a commercially available soil DNA extraction kit can be mentioned.

Furthermore, it is also possible to extract pathogenic fungi from diseased tissues of plant specimens, followed by separation cultivation, and to extract the DNA. For example, a diseased tissue is collected by using scissors or the like, the plant tissue piece obtained is surface-sterilized with hypochlorous acid or ethanol, placed in an exclusive medium or the like, and the fungi contained in the plant tissue is cultured.

It is also possible to collect airborne spores of fungi on agar medium by using an air sampler and to extract DNA after cultivation thereof.

In the above, as a medium used for cultivation of fungi, a common medium such as PDA (potato dextrose agar) may be used. In the case where fungi to be collected is determined in advance, the so-called selective medium to which antibiotics and the like are added may be used. As the culture conditions, it is preferable to leave the fungi at a temperature of 20°C to 25°C in a dark place for 65 hours or longer.

When cultivating fungi, next, cultured fungi colonies are collected for each colony individually or two or more colonies at once, and the colonies are frozen with liquid nitrogen or the like, and cells of the fungi contained in the colonies are crushed.

Then, genomic DNA is extracted from crushed cells of fungi thus obtained. Extraction of genomic DNA can be performed by a common method such as a method based on the CTAB method (Cetyl trimethyl ammonium bromide) or a method using a DNA extraction apparatus.

### (2) Amplification of target region

Next, the target region in the extracted genomic DNA is amplified. That is, a DNA fragment containing a target region in genomic DNA is amplified. The amplification method of this target region is not particularly limited, but a PCR method can be preferably used. In the PCR method, the target region is amplified by using a PCR reaction solution containing a primer set for amplifying the target region. As a PCR apparatus, a common thermal cycler or the like can be used.

By the method for detecting fungi according to the present embodiment, by conducting PCR under the following reaction conditions, for example, the target region of each fungi can be preferably amplified.
(a) 95°C 10 min, (b) 95°C (DNA denaturation step) 30 sec, (c) 56°C (annealing step) 30 sec, (d) 72°C (DNA synthesis step) 60 sec ((b) to (d) are repeated 40 cycles), (e) 72°C 10 minutes

As a reaction solution for PCR, for example, it is preferable to use one having the following composition. It is preferable to use a PCR reaction solution containing a nucleic acid synthesis substrate (dNTPmixture (dCTP, dATP, dTTP, dGTP)), a primer set, a nucleic acid synthesis enzyme (Nova Taq HotStart DNA polymerase, etc), a fluorescent labeling reagent (Cy5-dCTP, etc.), DNA of a sample, a buffer solution and water as a remaining component. As the buffer solution, Ampdirect (R) (manufactured by Shimadzu Corporation) can be used.

In this embodiment, the DNA fragments are amplified for the ITS region and the β-tubulin gene in the genomic DNA of fungi as the target region.

At this time, as a primer set for amplifying the ITS region, it is preferable to use a primer set comprising base sequences represented by SEQ ID NOS: 107 and 108 (SEQ ID NO: 107: forward primer, SEQ ID NO: 108: reverse primer) shown in Fig. 12.

As a primer set for amplifying the β-tubulin gene, it is preferable to use a primer set comprising base sequences represented by SEQ ID NOS: 109 and 110 (SEQ ID NO: 109: forward primer, SEQ ID NO: 110: reverse primer) shown in Fig. 12.

### (3) Confirmation of amplification product

Next, the amplification product is dropped on the carrier for detecting fungi according to the present embodiment, and the presence or absence of the amplification product is confirmed by detecting a label of the amplification product hybridized with the probe immobilized on the carrier for detecting fungi. In this way, it is possible to identify the fungi existing in the specimen.

Detection of the label can be carried out using a common label detection device such as a fluorescence scanning device or the like. For example, detection can be conducted by measuring the fluorescence intensity of the amplification product using BIOSHOT (R) of Toyo Kohan Co., Ltd. It is preferable to obtain the measurement results as S/N ratio (Signal to Noise ratio) value. The S/N ratio value is calculated by (median fluorescence intensity value - background value) ÷ background value. The label is not limited to fluorescence, and others can be used.

### [Kit for detecting fungi]

The kit for detecting fungi according to the present embodiment comprises a primer set to amplify a target region in DNA of fungi as a detection target, and a carrier for detecting fungi in which a probe for confirming the presence or absence of an amplification product is immobilized.

Specifically, it comprises a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110.

As the carrier for detecting fungi, one obtained by immobilizing a probe selected from each of a first probe group to detect Colletotrichum acutatum that comprises a probe, having a base sequence represented by SEQ ID NO: 101 selected from an ITS region and a probe having a base sequence represented by SEQ ID NO: 102 selected from β-tubulin genes, a second probe group to detect Fusarium solani that comprises a probe having a base sequence represented by SEQ ID NO: 103 selected from an ITS region, and a probe having a base sequence represented by SEQ ID NO: 104 selected from β-tubulin genes, a third probe group to detect Alternaria solani that comprises a probe to detect Alternaria solani having a base sequence represented by SEQ ID NO: 105 selected from an ITS region, and a fourth probe group to detect Rhizoctonia solani that comprises a probe having a base sequence represented by SEQ ID NO: 106 selected from an ITS region can be preferably used.

Further, the carrier may preferably one obtained by immobilizing all probes in the first probe group, the second probe group, the third probe group and the fourth probe group.

The kit for detecting fungi according to the present embodiment is used for detecting fungi by dropping an amplification product amplified with the use of the above-mentioned primer set to the carrier for detecting fungi, and by coupling the amplification product with a probe having a complementary base sequence.

According to the carrier for detecting fungi, the method for detecting fungi and the kit for detecting fungi according to the present embodiment, by using a probe capable of distinguishing four types of fungi mentioned above specifically, these fungi can be appropriately detected. Therefore, in soil environmental tests and the like, it is possible to simultaneously, rapidly and specifically with a high degree of accuracy detect these fungi, which are pathogenic fungi of plant diseases.

### EXAMPLES

### (Test 1)

By using the method for detecting microorganism, the kit for detecting microorganism and the microarray for detecting microorganism according to the present embodiment a test was conducted to examine whether microorganisms belonging to the genera Plasmodiophora, Pythium and Phytophthora can be detected individually with the use of primer sets and probes targeting a β-tubulin gene.

Specifically, by using a primer set which targets a β-tubulin gene and comprises a forward primer comprising a base sequence represented by SEQ ID NO: 1 and a reverse primer comprising a base sequence represented by SEQ ID NO: 4, β-tubulin genes in DNA of Plasmodiophora brassicae, Pythium aphanidermatum, Phytophthora infestans and Aspergillus versicolor were amplified independently by PCR.

Further, by using a primer set which targets a β-tubulin gene and comprises a forward primer comprising a base sequence represented by SEQ ID NO: 1 and a reverse primer comprising a base sequence represented by SEQ ID NO: 5, β-tubulin genes in DNA of Plasmodiophora brassicae, Pythium ultimaum, Phytophthora infestans and Aspergillus versicolor were amplified independently by PCR.

Then, to microarrays on which probes comprising base sequences represented by SEQ ID NOS: 12, 13, 15 and 17 were respectively immobilized, the amplification products as mentioned above were dropped the fluorescence label of the amplification products.

As for Plasmodiophora brassicae, that is an obligate parasite, those obtained from Laboratory of Plant Pathology, Kyoto Prefecture University were used. Microorganisms belonging to the genera Pythium and Phytophthora were obtained from NBRC (National Institute of Technology and Evaluation, NITE Biological Resource Center). The strains obtained from said center including those used in the tests mentioned later are as follows:
Pythium aphanidermatum NBRC7030
Pythium ultimum NBRC100125
Pythium zingiberis NBRC30818
Phytophthora infestans NBRC9174

As the PCR reaction solution, by using Ampdirect (R) (manufactured by Shimadzu Corporation), one having the following composition was used. As for each primer, one synthesized by Operon Technology Co., Ltd. was used.
- Ampdirect (G/Crich) 4.0 µl
- Ampdirect (addition-4) 4.0 µl
- dNTP Mixture (dATP, dCTP, dGTP, dTTP, 2.5 mM each) 1.0 µl
- Cy-5 dCTP 0.2 µl
- EX Taq Hot Start DNA polymerase (5U/µl) 0.2 µl
- Forward primer (10 µM) 1.0 µl
- Reverse primer (10 µM) 1.0 µl
- DNA of sample (100 pg/µl) 1.0 µl
- Water (added until the total volume became 20.0 µl)

Amplification of the gene by the PCR method was conducted by a nucleic acid amplification apparatus (TaKaRa PCR Thermal Cycler Dice (R) Gradient, manufactured by Takara Bio Inc.) under the same conditions described in the embodiment as follows.
(a) 95°C 10 sec
(b) 95°C 30 sec
(c) 56°C 30 sec
(d) 72°C 60 sec ((b) to (d) are repeated 40 cycles)
(e) 72°C 10 min

Next, a buffer solution (3 × SSC citric acid - physiological saline + 0.3% SDS) was mixed with the PCR amplification product, and the mixture was heated at 94° C for 5 minutes and added dropwise to the microarray. The microarray was allowed to stand at 45°C for 1 hour, and the PCR product not hybridized was washed away from the microarray using the buffer solution.

Then, the fluorescence intensity in each probe immobilized on the microarray (the fluorescence intensity of the amplification product coupled to the probe) was measured using a label detection apparatus (BIOSHOT (R), manufactured by Toyo Kohan Co., Ltd.) and the S/N ratio value of each probe was acquired. The results are shown in Fig. 3.

In Fig. 3, it is shown that that an amplification product obtained by amplifying a β-tubulin gene in DNA of Plasmodiophora brassicae by PCR using a primer set which comprises a forward primer comprising a base sequence represented by SEQ ID NO: 1 and a reverse primer comprising a base sequence represented by SEQ ID NO: 4 was coupled with probes comprising base sequences represented by SEQ ID NOS: 12 and 17, and labels were detected.

Further, it is shown that amplification products obtained by amplifying β-tubulin genes in DNA of Pythium aphanidermatum and Phytophthora infestans by PCR using a primer set which comprises a forward primer comprising a base sequence represented by SEQ ID NO: 1 and a reverse primer comprising a base sequence represented by SEQ ID NO: 4 were respectively coupled with were comprising base sequences represented by SEQ ID NOS: 13 and 17 and probes comprising base sequences represented by SEQ ID NOS: 15 and 17, and labels were detected.

Furthermore, it is shown that an amplification product obtained by amplifying a β-tubulin gene in DNA of Aspergillus versicolor by PCR using a primer set which comprises a forward primer comprising a base sequence represented by SEQ ID NO: 1 and a reverse primer comprising a base sequence represented by SEQ ID NO: 4 was coupled with a probe comprising a base sequence represented by SEQ ID NO: 17 and a label was detected.

Similarly, it is shown that an amplification product obtained by amplifying a β-tubulin gene in DNA of Plasmodiophora brassicae by PCR using a primer set which comprises a forward primer comprising a base sequence represented by SEQ ID NO: 1 and a reverse primer comprising a base sequence represented by SEQ ID NO: 5 was coupled with probes comprising base sequences represented by SEQ ID NOS: 12 and 17, and labels were detected.

Further, it is shown that amplification products obtained by amplifying β-tubulin genes in DNA of Pythium ultimum and Phytophthora infestans by PCR using a primer set which comprises a forward primer comprising a base sequence represented by SEQ ID NO: 1 and a reverse primer comprising a base sequence represented by SEQ ID NO: 5 were respectively coupled with probes comprising base sequences represented by SEQ ID NOS: 13 and 17 and probes comprising base sequences represented by SEQ ID NOS: 15 and 17, and labels were detected.

Furthermore, it is shown that an amplification product obtained by amplifying a β-tubulin gene in DNA of Aspergillus versicolor by PCR using a primer set which comprises a forward primer comprising a base sequence represented by SEQ ID NO: 1 and a reverse primer comprising a base sequence represented by SEQ ID NO: 5 was coupled with a probe comprising a base sequence represented by SEQ ID NO: 17, and a label was detected.

### (Test 2)

By using the method for detecting microorganism, the kit for detecting microorganism and the microarray for detecting microorganism according to the present embodiment, a further test was conducted to examine whether microorganisms belonging to the genera Plasmodiophora, Pythium and Phytophthora can be singly detected by using primer sets and probes targeting a β-tubulin gene.

Specifically, by using a primer set which targets a β-tubulin gene and comprises a forward primer comprising a base sequence represented by SEQ ID NO: 2 and a reverse primer comprising a base sequence represented by SEQ ID NO: 4, β-tubulin genes in DNA of Plasmodiophora brassicae, Pythium aphanidermatum, Phytophthora infestans and Aspergillus versicolor were amplified independently by PCR.

Further, by using a primer set which targets a β-tubulin gene and comprises a forward primer comprising a base sequence represented by SEQ ID NO: 2 and a reverse primer comprising a base sequence represented by SEQ ID NO: 5, β-tubulin genes in DNA of Plasmodiophora brassicae, Pythium zingiberis, Phytophthora infestans and Aspergillus versicolor were amplified independently by PCR.

Then, on microarrays on which probes comprising base sequences represented by SEQ ID NOS: 12, 14, 16 and 17 were respectively immobilized, the amplification products obtained by PCR as mentioned above were dropped to detect a fluorescence label of the amplification product.

Amplification of gene by the PCR reaction solution and the PCR method was conducted using the same composition under the same conditions as those in test 1.

Then, as in the same manner as in test 1, the PCR amplification product was hybridized with the probe, and the fluorescence intensity in each probe was measured using a label detection apparatus and the S/N ratio value of each probe was acquired. The results are shown in Fig. 4.

In Fig. 4, it is shown that an amplification product obtained by amplifying a β-tubulin gene in DNA of Plasmodiophora brassicae by PCR using a primer set which comprises a forward primer comprising a base sequence represented by SEQ ID NO: 2 and a reverse primer comprising a base sequence represented by SEQ ID NO: 4 was coupled with probes comprising base sequences represented by SEQ ID NOS: 12 and 17, and labels were detected.

Further, it is shown that amplification products obtained by amplifying β-tubulin genes in DNA of Pythium aphanidermatum and Phytophthora infestans by PCR using a primer set which comprises a forward primer comprising a base sequence represented by SEQ ID NO: 2 and a reverse primer comprising a base sequence represented by SEQ ID NO: 4 were coupled with probes comprising base sequences represented by SEQ ID NOS: 14 and 17 and base sequences represented by SEQ ID NOS: 16 and 17, and labels were detected.

Furthermore, it is shown that an amplification product obtained by amplifying a β-tubulin gene in DNA of Aspergillus versicolor by PCR using a primer set which comprises a forward primer comprising a base sequence represented by SEQ ID NO: 2 and a reverse primer comprising a base sequence represented by SEQ ID NO: 4 was coupled with a probe comprising a base sequence represented by SEQ ID NO: 17, and a label was detected.

Similarly, it is shown that an amplification product obtained by amplifying a β-tubulin gene in DNA of Plasmodiophora brassicae by PCR using a primer set which comprises a forward primer comprising a base sequence represented by SEQ ID NO: 2 and a reverse primer comprising a base sequence represented by SEQ ID NO: 5 was coupled with probes comprising base sequences represented by SEQ ID NOS: 12 and 17, and labels were detected.

Further, it is shown that amplification products obtained by amplifying β-tubulin genes in DNA of Pythium zingiberis and Phytophthora infestans by PCR using a primer set which comprises a forward primer comprising a base sequence represented by SEQ ID NO: 2 and a reverse primer comprising a base sequence represented by SEQ ID NO: 5 were respectively coupled with probes comprising a base sequences represented by SEQ ID NOS: 14 and 17 and probes comprising base sequences represented by SEQ ID NOS: 16 and 17, and labels were detected.

Furthermore, it is shown that an amplification product obtained by amplifying a β-tubulin gene in DNA of Aspergillus versicolor by PCR using a primer set which comprises a forward primer comprising a base sequence represented by SEQ ID NO: 2 and a reverse primer comprising a base sequence represented by SEQ ID NO: 5 was coupled with a probe comprising a base sequence represented by SEQ ID NO: 17, and a label was detected.

### (Test 3)

By using the method for detecting microorganism, the kit for detecting microorganism and the microarray for detecting microorganism according to the present embodiment, a further test was conducted to examine whether microorganisms belonging to the genera Plasmodiophora, Pythium and Phytophthora can be detected independently by using primer sets and probes targeting a β-tubulin gene.

Specifically, by using a primer set which targets a β-tubulin gene and comprises a forward primer comprising a base sequence represented by SEQ ID NO: 3 and a reverse primer comprising a base sequence represented by SEQ ID NO: 4, β-tubulin genes in DNA of Plasmodiophora brassicae, Pythium zingiberis, Phytophthora infestans and Aspergillus versicolor were amplified independently by PCR.

Further, by using a primer set which targets a β-tubulin gene and comprises a forward primer comprising a base sequence represented by SEQ ID NO: 3 and a reverse primer comprising a base sequence represented by SEQ ID NO: 5, β-tubulin genes in DNA of these four microorganisms were independently amplified by PCR.

On microarrays on which the probes comprising base sequences represented by SEQ ID NOS: 12, 14, 16 and 17 were respectively immobilized, the amplification products obtained by PCR mentioned above were dropped to detect a fluorescent label of the amplification product.

Amplification of gene by the PCR reaction solution and the PCR method was conducted using the same composition under the same conditions as those in test 1.

Then, as in the same manner as in test 1, the PCR amplification product was hybridized with the probe, and the fluorescence intensity in each probe was measured using a label detection apparatus and the S/N ratio value of each probe was acquired. The results are shown in Fig. 5.

In Fig. 5, it is shown that an amplification product obtained by amplifying a β-tubulin gene in DNA of Plasmodiophora brassicae by PCR using a primer set which comprises a forward primer comprising a base sequence represented by SEQ ID NO: 3 and a reverse primer comprising a base sequence represented by SEQ ID NO: 4 was coupled with probes comprising base sequences represented by SEQ ID NOS: 12 and 17, and labels were detected.

Further, it is shown that amplification products obtained by amplifying β-tubulin genes in DNA of Pythium zingiberis and Phytophthora infestans by PCR using a primer set which comprises a forward primer comprising a base sequence represented by SEQ ID NO: 3 and a reverse primer comprising a base sequence represented by SEQ ID NO: 4 were coupled with probes comprising base sequences represented by SEQ ID NOS: 14 and 17 and base sequences represented by SEQ ID NOS: 16 and 17, and labels were detected.

Furthermore, it is shown that an amplification product obtained by amplifying a β-tubulin gene in DNAof Aspergillus versicolor by PCR using a primer set which comprises a forward primer comprising a base sequence represented by SEQ ID NO: 3 and a reverse primer comprising a base sequence represented by SEQ ID NO: 4 was coupled with a probe comprising a base sequence represented by SEQ ID NO: 17, and a label was detected.

Similarly, it is shown that an amplification product obtained by amplifying a β-tubulin gene in DNA of Plasmodiophora brassicae by PCR using a primer set which comprises a forward primer comprising a base sequence represented by SEQ ID NO: 3 and a reverse primer comprising a base sequence represented by SEQ ID NO: 5 was coupled with probes comprising base sequences represented by SEQ ID NOS: 12 and 17, and labels were detected.

Further, it is shown that amplification products obtained by amplifying β-tubulin genes in DNA of Pythium zingiberis and Phytophthora infestans by PCR using a primer set which comprises a forward primer comprising a base sequence represented by SEQ ID NO: 3 and a reverse primer comprising a base sequence represented by SEQ ID NO: 5 were respectively coupled with probes comprising a base sequences represented by SEQ ID NOS: 14 and 17 and probes comprising base sequences represented by SEQ ID NOS: 16 and 17, and labels were detected.

Furthermore, it is shown that an amplification product obtained by amplifying a β-tubulin gene in DNAof Aspergillus versicolor by PCR using a primer set which comprises a forward primer comprising a base sequence represented by SEQ ID NO: 3 and a reverse primer comprising a base sequence represented by SEQ ID NO: 5 was coupled with a probe comprising a base sequence represented by SEQ ID NO: 17, and a label was detected.

### (Test 4)

By using the method for detecting microorganism, the kit for detecting microorganism and the microarray for detecting microorganism according to the present embodiment, a test was conducted to examine whether microorganisms belonging to the genera Plasmodiophora, Pythium and Phytophthora can be simultaneously detected by using primer sets and probes targeting a β-tubulin gene.

Specifically, by using a primer set which targets a β-tubulin gene and comprises a forward primer comprising a base sequence represented by SEQ ID NO: 1 and a reverse primer comprising a base sequence represented by SEQ ID NO: 4, β-tubulin genes in DNA of Plasmodiophora brassicae, Pythium aphanidermatum, Phytophthora infestans and Aspergillus versicolor were simultaneously amplified by PCR using a single PCR reaction solution.

Further, by using a primer set which targets a β-tubulin gene and comprises a forward primer comprising a base sequence represented by SEQ ID NO: 1 and a reverse primer comprising a base sequence represented by SEQ ID NO: 5, β-tubulin genes in DNA of Plasmodiophora brassicae, Pythium ultimum, Phytophthora infestans and Aspergillus versicolor were simultaneously amplified by PCR using a single PCR reaction solution.

Then, on microarrays on which probes comprising base sequences represented by SEQ ID NOS: 12, 13, 15 and 17 were respectively immobilized, the amplification products obtained by PCR mentioned above were dropped to detect a fluorescent label of the amplification product.

Amplification of gene by the PCR reaction solution and the PCR method was conducted using the same composition under the same conditions as those in test 1. However, as the PCR reaction solution, one containing 1.0 µl of the sample DNA per each type of microorganism (total 4.0 µl) was used.

Then, as in the same manner as in test 1, the PCR amplification products were hybridized with the probes, and the fluorescence intensity in each probe was measured using a label detection apparatus and the S/N ratio value of each probe was acquired. The results are shown in Fig. 6.

In Fig. 6, it is shown that amplification products obtained by amplifying β-tubulin genes in DNA of Plasmodiophora brassicaem, Pythium aphanidermatum and Phytophthora infestans by PCR using a primer set which comprises a forward primer comprising a base sequence represented by SEQ ID NO: 1 and a reverse primer comprising a base sequence represented by SEQ ID NO: 4 were respectively coupled with probes comprising base sequences represented by SEQ ID NOS: 12, 13 and 15, and labels were detected.

Further, it is shown that an amplification product obtained by amplifying a β-tubulin gene in DNA of Aspergillus versicolor by PCR using a primer set which comprises a forward primer comprising a base sequence represented by SEQ ID NO: 1 and a reverse primer comprising a base sequence represented by SEQ ID NO: 4 was coupled with a probe comprising a base sequence represented by SEQ ID NO: 17, and there is a possibility that a label was detected.

Similarly, it is shown that amplification products obtained by amplifying β-tubulin genes in DNA of Plasmodiophora brassicae, Pythium ultimum and Phytophthora infestans by PCR using a primer set which comprises a forward primer comprising a base sequence represented by SEQ ID NO: 1 and a reverse primer comprising a base sequence represented by SEQ ID NO: 5 were respectively coupled with probes comprising base sequences represented by SEQ ID NOS: 12, 13 and 15, and labels were detected.

Further, it is shown that an amplification product obtained by amplifying a β-tubulin gene in DNA of Aspergillus versicolor by PCR using a primer set which comprises a forward primer comprising a base sequence represented by SEQ ID NO: 1 and a reverse primer comprising a base sequence represented by SEQ ID NO: 5 was coupled with a probe comprising a base sequence represented by SEQ ID NO: 17, and there is a possibility that a label was detected.

### (Test 5)

By using the method for detecting microorganism, the kit for detecting microorganism and the microarray for detecting microorganism according to the present embodiment, a test was further conducted to examine whether microorganisms belonging to the genera Plasmodiophora, Pythium and Phytophthora can be simultaneously detected by using primer sets and probes targeting a β-tubulin gene.

Specifically, by using a primer set which targets a β-tubulin gene and comprises a forward primer comprising a base sequence represented by SEQ ID NO: 2 and a reverse primer comprising a base sequence represented by SEQ ID NO: 4, β-tubulin genes in DNA of Plasmodiophora brassicae, Pythium aphanidermatum, Phytophthora infestans and Aspergillus versicolor were simultaneously amplified by PCR using a single PCR reaction solution.

Further, by using a primer set which targets a β-tubulin gene and comprises a forward primer comprising a base sequence represented by SEQ ID NO: 2 and a reverse primer comprising a base sequence represented by SEQ ID NO: 5, β-tubulin genes in DNA of Plasmodiophora brassicae, Pythium zingiberis, Phytophthora infestans and Aspergillus versicolor were simultaneously amplified by PCR using a single PCR reaction solution.

Furthermore, by using a primer set which targets a β-tubulin gene and comprises a forward primer comprising a base sequence represented by SEQ ID NO: 3 and a reverse primer comprising a base sequence represented by SEQ ID NO: 4, β-tubulin genes in DNA of these four microorganisms were simultaneously amplified by PCR using a single PCR reaction solution.

In addition, by using a primer set which targets a β-tubulin gene and comprises a forward primer comprising a base sequence represented by SEQ ID NO: 3 and a reverse primer comprising a base sequence represented by SEQ ID NO: 5, β-tubulin genes in DNA of these four microorganisms were simultaneously amplified by PCR using a single PCR reaction solution.

Then, on microarrays on which probes comprising base sequences represented by SEQ ID NOS: 12, 14, 16 and 17 were respectively immobilized, the amplification products obtained by the PCR method mentioned above were dropped to detect a fluorescent label of the amplification product.

Amplification of gene by the PCR reaction solution and the PCR method was conducted using the same composition under the same conditions as those in test 1. However, as the PCR reaction solution, one containing 1.0 µl of the sample DNA per each type of microorganism (total 4.0µl) was used.

Then, as in the same manner as in test 1, the PCR amplification product was hybridized with the probe, and the fluorescence intensity in each probe was measured using a label detection apparatus and the S/N ratio value of each probe was acquired. The results are shown in Fig. 7.

In Fig. 7, it is shown that amplification products obtained by amplifying β-tubulin genes in DNA of Plasmodiophora brassicaem, Pythium aphanidermatum and Phytophthora infestans by PCR using a primer set which comprises a forward primer comprising a base sequence represented by SEQ ID NO: 2 and a reverse primer comprising a base sequence represented by SEQ ID NO: 4 were respectively coupled with probes comprising base sequences represented by SEQ ID NOS: 12, 14 and 16, and labels were detected.

Further, it is shown that an amplification product obtained by amplifying a β-tubulin gene in DNA of Aspergillus versicolor by PCR using a primer set which comprises a forward primer comprising a base sequence represented by SEQ ID NO: 2 and a reverse primer comprising a base sequence represented by SEQ ID NO: 4 was coupled with a probe comprising a base sequence represented by SEQ ID NO: 17, and there is a possibility that a label was detected.

Similarly, it is shown that amplification products obtained by amplifying β-tubulin genes in DNA of Plasmodiophora brassicae, Pythium zingiberis and Phytophthora infestans by PCR using a primer set which comprises a forward primer comprising a base sequence represented by SEQ ID NO: 2 and a reverse primer comprising a base sequence represented by SEQ ID NO: 5 were respectively coupled with probes comprising base sequences represented by SEQ ID NOS: 12, 14 and 16, and labels were detected.

Further, it is shown that an amplification product obtained by amplifying a β-tubulin gene in DNA of Aspergillus versicolor by PCR using a primer set which comprises a forward primer comprising a base sequence represented by SEQ ID NO: 2 and a reverse primer comprising a base sequence represented by SEQ ID NO: 5 was coupled with a probe comprising a base sequence represented by SEQ ID NO: 17, and there is a possibility that a label was detected.

Furthermore, it is shown that an amplification product obtained by amplifying β-tubulin genes in DNA of Plasmodiophora brassicae, Pythium zingiberis and Phytophthora infestans by PCR using a primer set which comprises a forward primer comprising a base sequence represented by SEQ ID NO: 3 and a reverse primer comprising a base sequence represented by SEQ ID NO: 4 were respectively coupled with probes comprising base sequences represented by SEQ ID NOS: 12, 14 and 16, and labels were detected.

In addition, it is shown that an amplification product obtained by amplifying a β-tubulin gene in DNA of Aspergillus versicolor by PCR using a primer set which comprises a forward primer comprising a base sequence represented by SEQ ID NO: 3 and a reverse primer comprising a base sequence represented by SEQ ID NO: 4 was coupled with a probe comprising a base sequence represented by SEQ ID NO: 17, and there is a possibility that a label was detected.

Similarly, it is shown that amplification products obtained by amplifying β-tubulin genes in DNA of Plasmodiophora brassicae, Pythium zingiberis and Phytophthora infestans by PCR using a primer set which comprises a forward primer comprising a base sequence represented by SEQ ID NO: 3 and a reverse primer comprising a base sequence represented by SEQ ID NO: 5 were respectively coupled with probes comprising base sequences represented by SEQ ID NOS: 12, 14 and 16, and labels were detected.

Further, it is shown that an amplification product obtained by amplifying a β-tubulin gene in DNA of Aspergillus versicolor by PCR using a primer set which comprises a forward primer comprising a base sequence represented by SEQ ID NO: 3 and a reverse primer comprising a base sequence represented by SEQ ID NO: 5 was coupled with a probe comprising a base sequence represented by SEQ ID NO: 17, and there is a possibility that a label was detected.

### (Test 6)

By using the method for detecting microorganism, the kit for detecting microorganism and the microarray for detecting microorganism according to the present embodiment, a test was conducted to examine whether microorganisms belonging to the genera Plasmodiophora, Pythium and Phytophthora can be independently detected by using primer sets and probes targeting an ITS region of rDNA gene.

Specifically, by using a primer set which targets an ITS region of rDNA gene and comprises a forward primer comprising a base sequence represented by SEQ ID NO: 6 and a reverse primer comprising a base sequence represented by SEQ ID NO: 9, ITS regions of rDNA gene in DNA of Plasmodiophora brassicae, Pythium aphanidermatum and Phytophthora infestans were amplified independently by PCR.

Further, by using a primer set which targets an ITS region of rDNA gene and comprises a forward primer comprising a base sequence represented by SEQ ID NO: 7 and a reverse primer comprising a base sequence represented by SEQ ID NO: 10, ITS regions of rDNA gene in DNA of these three types of microorganism were independently amplified by PCR.

Furthermore, by using a primer set which targets an ITS region of rDNA gene and comprises a forward primer comprising a base sequence represented by SEQ ID NO: 8 and a reverse primer comprising a base sequence represented by SEQ ID NO: 10, ITS regions of rDNA gene in DNA of these three microorganisms were independently amplified by PCR.

In addition, by using a primer set which targets an ITS region of rDNA gene and comprises a forward primer comprising a base sequence represented by SEQ ID NO: 8 and a reverse primer comprising a base sequence represented by SEQ ID NO: 11, ITS regions of rDNA gene in DNA of these three types of microorganism were independently amplified by PCR.

Then, on microarrays on which the probes comprising base sequences represented by SEQ ID NO: 18, 19, 20 and 21 were respectively immobilized, the amplification products obtained by the PCR method as mentioned above were dropped to detect a fluorescent label of the amplification product.

Amplification of genes by the PCR reaction solution and the PCR method was conducted using the same composition under the same conditions as those in test 1.

Then, as in the same manner as in test 1, the PCR amplification product was hybridized with the probe, and the fluorescence intensity in each probe was measured using a label detection apparatus and the S/N ratio value of each probe was acquired. The results are shown in Fig. 9.

In Fig. 9, it is shown that an amplification product obtained by amplifying an ITS region of rDNA gene in DNA of Plasmodiophora brassicae by PCR using a primer set which comprises a forward primer comprising a base sequence represented by SEQ ID NO: 6 and a reverse primer comprising a base sequence represented by SEQ ID NO: 9 was coupled with a probe comprising a base sequence represented by SEQ ID NO: 18, and a label was detected.

Further, it is shown that an amplification product obtained by amplifying an ITS region of rDNA gene in DNA of Pythium ultimum by PCR using a primer set which comprises a forward primer comprising a base sequence represented by SEQ ID NO: 7 and a reverse primer comprising a base sequence represented by SEQ ID NO: 10 was coupled with a probe comprising a base sequence represented by SEQ ID NO: 19 and a label was detected.

Furthermore, it is shown that an amplification product obtained by amplifying an ITS region of rDNA gene in DNA of Pythium ultimum by PCR using a primer set which comprises a forward primer comprising a base sequence represented by SEQ ID NO: 8 and a reverse primer comprising a base sequence represented by SEQ ID NO: 10 was coupled with a probe comprising a base sequence represented by SEQ ID NO: 19 and a label was detected.

In addition, it is shown that amplification products obtained by amplifying ITS regions of rDNA gene in DNA of Pythium ultimum and Phytophthora infestans by PCR using a primer set which comprises a forward primer comprising a base sequence represented by SEQ ID NO: 8 and a reverse primer comprising a base sequence represented by SEQ ID NO: 11 were coupled with probes comprising base sequences represented by SEQ ID NOS: 20 and 21 and labels were detected.

### (Test 7)

By using the method for detecting microorganism, the kit for detecting microorganism and the microarray for detecting microorganism according to the present embodiment, a test was conducted to examine whether microorganisms belonging to the genera Plasmodiophora, Pythium and Phytophthora can be simultaneously detected by using primer sets and probes targeting an ITS region of rDNA gene.

Specifically, by using a primer set which targets an ITS region of rDNA gene and comprises a forward primer comprising a base sequence represented by SEQ ID NO: 6 and a reverse primer comprising a base sequence represented by SEQ ID NO: 9 and which targets an ITS region of rDNA gene and comprises a forward primer comprising a base sequence represented by SEQ ID NO: 7 and a reverse primer comprising a base sequence represented by SEQ ID NO. 10, ITS regions of rDNA gene in DNA of Plasmodiophora brassicae and Pythium ultimum were amplified simultaneously by PCR using a single PCR reaction solution.

Further, by using a primer set which targets an ITS region of rDNA gene and comprises a forward primer comprising a base sequence represented by SEQ ID NO: 6 and a reverse primer comprising a base sequence represented by SEQ ID NO: 9 and a primer set which targets an ITS region of rDNA gene and comprises a forward primer comprising a base sequence represented by SEQ ID NO: 8 and a reverse primer comprising a base sequence represented by SEQ ID NO. 11, ITS regions of rDNA gene in DNA of Plasmodiophora brassicae and Pythium ultimum were amplified simultaneously by PCR using a single PCR reaction solution.

Furthermore, by using a primer set which targets an ITS region of rDNA gene and comprises a forward primer comprising a base sequence represented by SEQ ID NO: 6 and a reverse primer comprising a base sequence represented by SEQ ID NO: 9 and a primer set which targets an ITS region of rDNA gene and comprises a forward primer comprising a base sequence represented by SEQ ID NO: 8 and a reverse primer comprising a base sequence represented by SEQ ID NO. 11, ITS regions of rDNA gene in DNA of Plasmodiophora brassicae and Phytophthora infestans were amplified simultaneously by PCR using a single PCR reaction solution.

Then, on microarrays on which probes comprising base sequences represented by SEQ ID NOS: 18, 19, 20 and 21 were respectively immobilized, the amplification products obtained by the PCR method mentioned above were dropped to detect a fluorescent label of the amplification product.

Amplification of gene by the PCR reaction solution and the PCR method was conducted using the same composition under the same conditions as those in test 1. However, as the PCR reaction solution, one containing 1.0 µl of the sample DNA per each type of microorganism (total 2.0 µl) was used.

Then, as in the same manner as in test 1, the PCR amplification product was hybridized with the probe, and the fluorescence intensity in each probe was measured using a label detection apparatus and the S/N ratio value of each probe was acquired. The results are shown in Fig. 10.

In Fig. 10, it is shown that amplification products obtained by amplifying ITS regions of rDNA gene in DNA of Plasmodiophora brassicae and Pythium ultimum by PCR using a primer set which comprises a forward primer comprising a base sequence represented by SEQ ID NO: 6 and a reverse primer comprising a base sequence represented by SEQ ID NO: 9 and a primer set which comprises a forward primer comprising a base sequence represented by SEQ ID NO: 7 and a reverse primer comprising a base sequence represented by SEQ ID NO: 10 were respectively coupled with probes comprising base sequences represented by SEQ ID NOS: 18 and 19, and label were detected.

Further, it is shown that an amplification product obtained by amplifying ITS regions of rDNA gene in DNA of Plasmodiophora brassicae and Pythium ultimum by PCR using a primer set which comprises a forward primer comprising a base sequence represented by SEQ ID NO: 6 and a reverse primer comprising a base sequence represented by SEQ ID NO: 9 and a primer set which comprises a forward primer comprising a base sequence represented by SEQ ID NO: 8 and a reverse primer comprising a base sequence represented by SEQ ID NO: 11 were coupled with probes comprising base sequences represented by SEQ ID NO: 18, 20 and 21, and label were detected.

Furthermore, it is shown that an amplification product obtained by amplifying ITS regions of rDNA gene in DNA of Plasmodiophora brassicae and Phytophthora infestans by PCR using a primer set which comprises a forward primer comprising a base sequence represented by SEQ ID NO: 6 and a reverse primer comprising a base sequence represented by SEQ ID NO: 9 and a primer set which comprises a forward primer comprising a base sequence represented by SEQ ID NO: 8 and a reverse primer comprising a base sequence represented by SEQ ID NO: 11 was coupled with probes comprising base sequences represented by SEQ ID NO: 18, 20 and 21 and label were detected.

Hereinbelow, a specific explanation will be made on a test conducted for confirming the effects of the carrier for detecting fungi, the method for detecting fungi and the kit for detecting fungi according to the embodiment of the present invention.

### [Test 8]

As the carrier for detecting fungi, Gene Silicon (R) (manufactured by Toyo Kohan Co., Ltd.) immobilizing probes comprising base sequences represented by SEQ ID NOS: 101 to 106 shown Fig. 11 was used. As for each probe, one synthesized by Operon Technology Co., Ltd. was used. Immobilizing on the substrate of each probe was conducted by using a microarray device.

For the fungi to be detected, a strain obtained from National Institute of Technology and Evaluation, Biotechnology Headquarters (NITE Biological Resource Center) was used. Specifically, the following four kinds of strains were used.
(1) Colletotrichum acutatum NBRC32850
(2) Fusarium solani NBRC7707
(3) Alternaria solani NBRC7516
(4) Rhizoctonia solani NBRC30984

These fungi were cultured for each strain. Cultivation was carried out by using a PDA medium and by allowing to stand at 25°C in the dark for 7 days.

Further, cultured fungi colonies were collected, individually placed in a vial containing ϕ 0.5 mm zirconia beads, immersed in liquid nitrogen to freeze the sample, and then, the fungi cells were crushed by using a shaking device.

Next, genomic DNA of fungi were extracted by a DNA extraction device, and an ITS region and a β-tubulin gene of each fungi (as for (3) and (4), only an ITS region was actually amplified) were amplified for each strain.

At this time, as a primer set for amplifying the ITS region, a forward primer comprising a base sequence represented by SEQ ID NO: 107 shown in FIG. 12 and a reverse primer comprising a base sequence represented by SEQ ID NO: 108 were used. Further, as a primer set for amplifying a β-tubulin gene, a forward primer comprising a base sequence represented by SEQ ID NO: 109 and a reverse primer comprising a base sequence represented by SEQ ID NO: 110 shown in Fig. 12 was used.

As for the above-mentioned primers, those synthesized by Operon Technology Co., Ltd. were used.

As the reaction solution for PCR, by using Ampdirect (R) (manufactured by Shimadzu Corporation), a PCR reaction solution having the following composition was prepared in a quantity of 20 µl for each strain.
1. Ampdirect (G/Crich) 4.0 µl
2. Ampdirect (addition-4) 4.0 µl
3. dNTPmix 1.0 µl
4. Cy-5dCTP 0.2 µl
5. ITS1-Fw primer (SEQ ID NO: 107) (2.5 µM) 1.0 µl
6. ITS1-Rv primer (SEQ ID NO: 108) (2.5 µM) 1.0 µl
7. BtF primer (SEQ ID NO: 109) (10 µM) 1.0 µl
8. BtR primer (SEQ ID No: 110) (10 µM) 1.0 µl
9. Template DNA 1.0 µl (100 pg/µl)
10. NovaTaq HotStart DNA polymerase 0.2 µl
11. Water (added until the entire quantity became 20.0 µl)

By a nucleic acid amplification device (TaKaRa PCR Thermal Cycler Dice (R), manufactured by TAKARA BIO Inc.) with the use of the above-mentioned PCR reaction solution, amplification of DNA was conducted under the following conditions.
(a) 95°C 10 min
(b) 95°C 30 sec
(c) 56°C 30 sec
(d) 72°C 60 sec ((b) to (d) are repeated 40 cycles)
(e) 72°C 10 min

Subsequently, the PCR amplification product was mixed with a buffer solution (3 × SSC citric acid-physiological saline + 0.3% SDS), the mixture was heated at 94°C for 5 minutes. The mixture was added dropwise to the carrier for detecting fungi (DNA chip) mentioned above. This carrier for detecting fungi was allowed to stand at 45° C for 1 hour, and the PCR product which was not hybridized was washed out from the DNA chip by using the above-mentioned buffer solution.

Then, a fluorescence intensity in each probe immobilized on the carrier for detecting fungi (the fluorescence intensity of the amplification product coupled with the probe) was measured using a label detection apparatus (BIOSHOT (R), manufactured by Toyo Kohan Co., Ltd.), and a S/N ratio value in each probe was acquired.

As mentioned above, the target region in DNA of each of Colletotrichum acutatum, Fusarium solani, Alternaria solani and Rhizoctonia solani was individually amplified, and the resulting amplification product was hybridized with each probe. As a result, a S/N ratio value in probes comprising base sequences represented by SEQ ID NOS: 101 to 106 are as shown in Fig. 13.

That is, when a sample fungus (detection target fungus) is Colletotrichum acutatum, in probes comprising base sequences represented by SEQ ID NOS: 101 and 102 for detecting Colletotrichum acutatum, the S/N ratio value is 3 or more. Hence it can be judged to be positive. Further, in probes comprising base sequences represented by SEQ ID NOS: 103 to 106 for detecting other types of fungi, the S/N ratio was less than 3.-Hence it can be judged to be negative. Therefore, it can be understood that these probes effectively function to detect Colletotrichum acutatum.

Further, when a sample fungus is Fusarium solani, in probes comprising base sequences represented by SEQ ID NOS: 103 and 104 for detecting Fusarium solani, the S/N ratio value is 3 or more. Hence it can be judged to be positive. Further, in probes comprising base sequences represented by SEQ ID NOS: 101, 102, 105 and 106 for detecting other types of fungi, the S/N ratio was less than 3. Hence it can be judged to be negative. Therefore, it can be understood that these probes effectively function to detect Fusarium solani.

Furthermore, when a sample fungus is Alternaria solani, in a probe comprising a base sequence represented by SEQ ID NO: 105 for detecting Alternaria solani, the S/N ratio value is 3 or more. Hence it can be judged to be positive. Further, in probes comprising base sequences represented by SEQ ID NOS: 101 to 104 and 106 for detecting other types of fungi, the S/N ratio was less than 3. Hence it can be judged to be negative. Therefore, it can be understood that these probes effectively function to detect Alternaria solani.

In addition, when a sample fungus is Rhizoctonia solani, in a probe comprising a base sequence represented by SEQ ID NO: 106 for detecting Rhizoctonia solani, the S/N ratio value is 3 or more. Hence it can be judged to be positive. Further, in probes comprising base sequences base sequences represented by SEQ ID NOS: 101 to 105 for detecting other types of fungi, the S/N ratio was less than 3. Hence it can be judged to be negative. Therefore, it can be understood that these probes effectively function to detect Rhizoctonia solani.

### [Test 9]

A test was conducted to confirm whether a probe immobilized on the carrier for detecting fungi according to the present embodiment functioned effectively when a multiplex PCR was conducted for simultaneously amplifying target regions in DNA of plural types of target fungi.

As the carrier for detecting fungi, the same one that used in test 8 was used. As for the target fungi, four types of strain as those in test 8 were used.

In this test, Colletotrichum acutatum, Fusarium solani, Alternaria solani and Rhizoctonia solani were respectively cultured by using a PDA medium and by allowing to stand at 25°C in the dark for 7 days.

Further, colonies were collected from each cultured fungus and composed, and the composed sample comprising these four types of fungi were placed in a vial containing ϕ 0.5 mm zirconia beads, immersed in liquid nitrogen to freeze the sample, and then, the fungi cells were crushed by using a shaking device.

Next, genomic DNA of fungi were extracted by a DNA extraction device, and an ITS region and a β-tubulin gene of each fungi (as for Alternaria solani and Rhizoctonia solani, only ITS region was actually amplified) were simultaneously amplified. The primer sets used in the PCR method are the same as that used in test 8.

As the reaction solution for PCR, by using Ampdirect (R) (manufactured by Shimadzu Corporation), a PCR reaction solution having the following composition was prepared in a quantity of 20 µl.
1. Ampdirect (G/Crich) 4.0 µl
2. Ampdirect (addition-4) 4.0 µl
3. dNTPmix 1.0 µl
4. Cy-5dCTP 0.2 µl
5. ITS1-Fw primer (2.5 µM) 1.0µl
6. ITS1-Rv primer (2.5 µM) 1.0µl
7. BtF primer (10 µM) 1.0 µl
8. BtR primer (10 µM) 1.0 µl
9. Template DNA(1.0 µl/strain)(100 pg/µl) 1.0µl×4
10. NovaTaq HotStart DNA polymerase 0.2 µl
11. Water (added until the entire quantity became 20.0 µl)

Amplification of DNA was conducted by a nucleic acid amplification apparatus (TaKaRa PCR Thermal Cycler Dice (R) Gradient Takara Bio Inc.) with the use of the above-mentioned PCR reaction solution under the same conditions as those in test 8.

Next, a buffer solution (3 × SSC citric acid - physiological saline + 0.3% SDS) was mixed with the PCR amplification product, and the mixture was heated at 94° C for 5 minutes and added dropwise to the carrier for detecting fungi (DNA chip). The carrier for detecting fungi was allowed to stand at 45°C for 1 hour, and the PCR product not hybridized was washed away from the DNA chip using the buffer solution.

Then, a fluorescence intensity in each probe immobilized on the carrier for detecting fungi was measured using a label detection apparatus (GenePix4100A manufactured by Molecular Devices, LLC), and a S/N ratio value in each probe was acquired. The results are shown in Fig. 13.

As mentioned above, target regions in DNA of Colletotrichum acutatum, Fusarium solani, Alternaria solani and Rhizoctonia solani were simultaneously amplified by using single PCR reaction solution, and the resulting amplification product was hybridized with each probe. As a result, in a probe comprising a base sequence represented by SEQ ID NO: 101 for detecting Colletotrichum acutatum, the S/N ratio value was 3 or more, and in a probe comprising a base sequence represented by SEQ ID NO: 102 for detecting Colletotrichum acutatum, the S/N ratio value was less than 3. From these results, it can be judged that a positive reaction was obtained for Colletotrichum acutatum.

In probes for detecting Fusarium solani which comprises base sequences represented by SEQ ID NOS: 103 and 104, the S/N ratio value was 3 or more, and hence judged to be positive for Fusarium solani.

In a probe for detecting Alternaria solani which comprises a base sequence represented by SEQ ID NO: 105, the S/N ratio value was 3 or more, and hence judged to be positive for Alternaria solani.

In a probe for detecting Rhizoctonia solani which comprises a base sequence represented by SEQ ID NO: 106, the S/N ratio value was 3 or more, and hence judged to be positive for Rhizoctonia solani.

Accordingly, it was confirmed that a probe immobilized on the carrier for detecting fungi according to the present embodiment effectively functioned when multiplex PCR was conducted for simultaneously amplifying a target region in DNA of a plural types of detection target fungi.

### [Test 10]

A test was conducted for confirming that probes immobilized on the carrier for detecting fungi according to the present embodiment were not hybridized with amplification products of target regions in DNA of fungi other than the fungi as the detection target.

As the carrier for detecting fungi, the same as that used in test 8 was used.

As fungi other than detection targets, strains obtained from the Japan Collection of Microorganisms of RIKEN BioResource Center, RIKEN and from the Biological Resource Center of the National Institute of Technology and Evaluation (NITE) were used. Specifically, strains of the following six types of fungi were used.
(1) Aspergillus flavus JCM10252
(2) Chaetomium globosum NBRC6347
(3) Curvularia lunata NBR100165
(4) Eurotium repens JCM1580
(5) Penicillium digitatum JCM9863
(6) Phoma destructiva NBRC7482

These fungi were cultured for each strain. Cultivation was carried out by using a PDA medium and by allowing to stand at 25°C in the dark for 7 days.

Further, cultured fungi colonies were collected, individually placed in a vial containing ϕ 0.5 mm zirconia beads, immersed in liquid nitrogen to freeze the sample, and then, the fungi cells were crushed by using a shaking device.

Next, genomic DNA of fungi was extracted by a DNA extraction device, and an amplification reaction of an ITS region and/or a β-tubulin gene of each fungi was conducted for each strain by the PCR method in the same manner as in the test 8.

Next, a buffer solution (3 × SSC citric acid - physiological saline + 0.3% SDS) was mixed with the PCR amplification product, and the mixture was heated at 94°C for 5 minutes and added dropwise to the above-mentioned carrier for detecting fungi. The carrier for detecting fungi was allowed to stand at 45°C for 1 hour, and the PCR product not hybridized was washed away from the microarray using the buffer solution.

Then, a fluorescence intensity in each probe immobilized on the carrier for detecting fungi was measured using a label detection apparatus (BIOSHOT (R), manufactured by Toyo Kohan Co., Ltd.) and the S/N ratio value of each probe was acquired. The results are shown in Fig. 13.

As mentioned above, amplification reaction of the target region in DNA of Aspergillus flavus, Ketomium globosum, Carbara lunata, Eurotium repense, Penicillium digitatum, and Formal destructiva was performed, and the resulting amplification products were hybridized with each probe. As a result, all S/N ratio values in probes comprising base sequences represented by SEQ ID NOS: 101 to 106 were less than 3, and judged to be negative. Therefore, each probe in the carrier for detecting fungi according to the present embodiment did not show a false positive reaction, and hence it is understood it effectively functions.

The present invention is not restricted to the embodiment and the Examples mentioned above, and various modifications are possible within the scope of the invention.

For example, each of the above-mentioned primer sets may be used singly in a PCR reaction solution, or a plurality of primer sets may be simultaneously used in a PCR reaction solution in all combinations. Further, in a microarray for detecting microorganism, each of the above-mentioned probes may be used singly or plurally for any combinations. As mentioned above, appropriate modifications are possible.

Further, for example, in the carrier for detecting fungi according to the present embodiment, each probe may be immobilized one by one or in plural spots. Further, other probes for detecting fungi other than the fungi as the detection target in the present embodiment can be immobilized together with the probe according to the present embodiment.

### Industrial Applicability

The present invention can be preferably utilized in a test for plant tissue and soil, epidemiological environmental test, environmental test, clinical test, animal health etc.

Further, the present invention can preferably be used when specifically and with multiplicity Colletotrichum acutatum, Fusarium solani, Alternaria solani and Rhizoctonia solani which are plant pathogenic fungi.

## Claims

1. A method for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
wherein the PCR is conducted, for a β-tubulin gene in DNA of microorganisms belonging to the genus Plasmodiophora as the PCR amplification target, with the use of the extracted DNA and a first primer set which comprises a forward primer comprising at least one of base sequences represented by SEQ ID NOS: 1-3 and a reverse primer comprising at least one of base sequences represented by SEQ ID NOS: 4 and 5, and then the presence or absence of the microorganisms belonging to the genus Plasmodiophora in the sample is determined on the basis of the obtained amplification product.

2. The method for detecting microorganism according to claim 1, wherein a first amplification product obtained through the PCR with the use of DNA of microorganisms belonging to the genus Plasmodiophora and the first primer set is brought into contact with a microarray immobilizing a first probe, which comprises at least one of a base sequence represented by SEQ ID NO: 12 and a complementary sequence of said base sequence represented by SEQ ID NO:12, to be complementarily coupled with the first amplification product, and
a label of the first amplification product complementarily coupled with the first probe is detected.

3. The method for detecting microorganism according to claim 1 or 2, wherein the PCR is conducted, for a β-tubulin gene in DNA of microorganisms belonging to the genera Plasmodiophora, Pythium and Phytophthora as PCR amplification targets, with the use of the first primer set and the extracted DNA, and
the presence or absence of microorganisms belonging to the genera Plasmodiophora, Pythium and Phytophthora in the sample is simultaneously determined on the basis of the obtained amplification product.

4. The method for detecting microorganism according to claim 3, wherein a second amplification product obtained through the PCR with the use of DNA of microorganisms belonging to the genus Pythium and the first primer set is brought into contact with a microarray immobilizing a second probe, which comprises at least one of base sequences represented by SEQ ID NOS: 13 and 14 and complementary sequences of said base sequences represented by SEQ ID NS: 13 and 14, to be complementarily coupled with the second amplification product, and
a label of the second amplification product complementarily coupled with the second probe is detected.

5. The method for detecting microorganism according to claim 3 or 4, wherein a third amplification product obtained through the PCR with the use of DNA of microorganisms belonging to the genus Phytophthora and the first primer set is brought into contact with a microarray immobilizing a third probe, which comprises at least one of base sequences represented by SEQ ID NOS: 15 and 16, to be complementarily coupled with the third amplification product, and
a label of the third amplification product complementarily coupled with the third probe is detected.

6. The method for detecting microorganism according to any one of claims 3 to 5, wherein a fourth amplification product obtained through the PCR with the use of DNA of at least one of the microorganisms belonging to the genera Plasmodiophora, Pythium and Phytophthora and other microorganisms and the first primer set is brought into contact with a microarray immobilizing a fourth probe, which comprises at least one of a base sequence represented by SEQ ID NO: 17 and a complementary sequence of said base sequence represented by SEQ ID NO: 17, to be complementarily coupled with a fourth amplification product, and
a label of the fourth amplification product complementarily coupled with the fourth probe is detected.

7. The method for detecting microorganism according to any one of claims 1 to 6, wherein the PCR is conducted, for an ITS region of rDNA gene in DNA of microorganisms belonging to the genus Plasmodiophora as the PCR amplification target, with the use of the extracted DNA and a second primer set which comprises a forward primer comprising a base sequence represented by SEQ ID NO: 6 and a reverse primer comprising a base sequence represented by SEQ ID NO: 9, and
the presence or absence of the microorganisms belonging to the genus Plasmodiophora in the sample is determined on the basis of the obtained amplification product.

8. The method for detecting microorganism according to claim 7, wherein a fifth amplification product obtained through the PCR with the use of DNA of microorganisms belonging to the genus Plasmodiophora and the second primer set is brought into contact with a microarray immobilizing a fifth probe, which comprises at least one of a base sequence represented by SEQ ID NO: 18 and a complementary sequence of the base sequence represented by SEQ ID NO: 18, to be complementarily coupled with the fifth amplification product, and
a label of the fifth amplification product complementarily coupled with the fifth probe is detected.

9. The method for detecting microorganism according to any one of claims 1 to 8, wherein the PCR is conducted, for an ITS region of rDNA gene in DNA of microorganisms belonging to the genus Pythium as the PCR amplification target with the use of the extracted DNA and a third primer set which comprises a forward primer comprising at least one of base sequences represented by SEQ ID NOS: 7 and 8 and a reverse primer comprising a base sequence represented by SEQ ID NO: 10, and
the presence or absence of the microorganisms belonging to the genus Pythium in the sample is determined on the basis of the obtained amplification product.

10. The method for detecting microorganism according to claim 9, wherein a sixth amplification product obtained through the PCR with the use of DNA of microorganisms belonging to the genus Pythium and the third primer set is brought into contact with a microarray immobilizing a sixth probe, which comprises at least one of a base sequence represented by SEQ ID NO: 19 and a complementary sequence of said base sequence represented by SEQ ID NO: 19, to be complementarily coupled with the sixth amplification product, and
a label of the sixth amplification product complementarily coupled with the sixth probe is detected.

11. The method for detecting microorganism according to any one of claims 1 to 10, wherein the PCR is conducted, for an ITS region of rDNA gene in DNA of microorganisms belonging to the genus Pythium or Phytophthora as the PCR amplification target, with the use of the extracted DNA and a fourth primer set which comprises a forward primer comprising a base sequence represented by SEQ ID NO: 8 and a reverse primer comprising a base sequence represented by SEQ ID NO: 11, and
the presence or absence of the microorganisms belonging to the genus Pythium or Phytophthora in the sample is determined on the basis of the obtained amplification product.

12. The method for detecting microorganism according to claim 11, wherein a seventh amplification product obtained through the PCR with the use of DNA of microorganisms belonging to the genus Pythium or Phytophthora and the fourth primer set is brought into contact with a microarray immobilizing a seventh probe, which comprises at least one of base sequences represented by SEQ ID NOS: 20 and 21 and complementary sequences of at least one of the base sequences represented by SEQ ID NOS: 20 and 21, to be complementarily coupled with the seventh amplification product, and
a label of the seventh amplification product complementarily coupled with the seventh probe is detected.

13. A kit for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microorganisms test kit being **characterized by** comprising a primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for a β-tubulin gene in DNA of microorganisms belonging to the genera Plasmodiophora, Pythium and Phytophthora.

14. The kit for detecting microorganism according to claim 13, which further includes a primer set which comprises a forward primer comprising a base sequence represented by SEQ ID NO: 6 and a reverse primer comprising a base sequence represented by SEQ ID NO: 9 as targets of amplification by the PCR for an ITS region of rDNA gene in DNA of microorganisms belonging to the genus Plasmodiophora.

15. The kit for detecting microorganism according to claim 13 or 14, which further includes a primer set which comprises a forward primer comprising any one of base sequences represented by SEQ ID NOS: 7 and 8 and a reverse primer comprising a base sequence represented by SEQ ID NO: 10 as targets of amplification by the PCR for an ITS region of rDNA gene in DNA of microorganisms belonging to the genus Pythium.

16. The kit for detecting microorganism according to any one of claims 13 to 15, which further includes a primer set which comprises a forward primer comprising a base sequence represented by SEQ ID NO: 8 and a reverse primer comprising a base sequence represented by SEQ ID NO: 11 as targets of amplification by the PCR for an ITS region of rDNA gene in DNA of microorganisms belonging to the genus Pythium or Phytophthora.

17. A microarray for detecting microorganism which comprises collecting a sample from a plant tissue, soil, water or any other environment, extracting DNA from microorganisms contained in the sample, conducting PCR with the use of the extracted DNA, and determining the presence or absence of the microorganisms in the sample on the basis of an obtained amplification product,
the microarray being **characterized by** immobilizing a first probe, which comprises at least one of a base sequence represented by SEQ ID NO: 12 and a complementary sequence of said base sequence represented by SEQ ID NO: 12, to be complementarily coupled with a first amplification product obtained by PCR with the use of a first primer set which comprises a forward primer comprising at least one of base sequences represented by SEQ ID NOS: 1-3 and a reverse primer comprising at least one of base sequences represented by SEQ ID NOS: 4 and 5 as targets of amplification by the PCR for DNA of microorganisms belonging to the genus Plasmodiophora and a β-tubulin gene in DNA of microorganisms belonging to the genus Plasmodiophora.

18. The microarray for detecting microorganism according to claim 17 which is **characterized by** further immobilizing a second probe, which comprises at least one of base sequences represented by SEQ ID NOS: 13 and 14 and complementary sequences of said base sequences represented by SEQ ID NOS: 13 and 14, to be complementarily coupled with a second amplification product obtained through the PCR with the use of DNA of microorganisms belonging to the genus Pythium and the first primer set.

19. The microarray for detecting microorganism according to claim 17 or 18 which is **characterized by** further immobilizing a third probe, which comprises at least one of base sequences represented by SEQ ID NOS: 15 and 16, to be complementarily coupled with a third amplification product obtained through the PCR with the use of DNA of microorganisms belonging to the genus Phytophthora and the first primer set

20. The microarray for detecting microorganism according to any one of claims 17 to 19 which is **characterized by** further immobilizing a fourth probe, which comprises at least one of a base sequence represented by SEQ ID NO: 17 and a complementary sequence of said base sequence represented by SEQ ID NO: 17, to be complementarily coupled with a fourth amplification product obtained through the PCR with the use of DNA of at least one of microorganisms belonging to the genera Plasmodiophora, Pythium, Phytophthora and other microorganisms and the first primer set.

21. The microarray for detecting microorganism according to any one of claims 17 to 20 which is **characterized by** further immobilizing a fifth probe, which comprises at least one of a base sequence represented by SEQ ID NO: 18 and complementary sequence of said base sequence represented by SEQ ID NO: 18, to be complementarily coupled with a fifth amplification product obtained by the PCR with the use of a second primer set which comprises a forward primer comprising a base sequence represented by SEQ ID NO: 6 and a reverse primer comprising a base sequence represented by SEQ ID NO: 9 as targets of amplification by the PCR for DNA of microorganisms belonging to the genus Plasmodiophora and an ITS region of rDNA gene in DNA of microorganisms belonging to the genus Plasmodiophora.

22. The microarray for detecting microorganism according to any one of claims 17 to 21 which is **characterized by** further immobilizing a sixth probe, which comprises at least one of a base sequence represented by SEQ ID NO: 19 and a complementary sequence of said base sequence represented by SEQ ID NO: 19, to be complementarily coupled with a sixth amplification product obtained by the PCR with the use of a third primer set which consists of a forward primer comprising at least one of base sequences represented by SEQ ID NOS: 7 and 8 and a reverse primer comprising a base sequence represented by SEQ ID NO: 10 as targets of amplification by the PCR for DNA of microorganisms belonging to the genus Pythium and an ITS region of rDNA gene in DNA of microorganisms belonging to the genus Pythium.

23. The microarray for detecting microorganism according to any one of claims 17 to 22 which is **characterized by** further immobilizing a seventh probe, which comprises at least one of base sequences represented by SEQ ID NOS: 20 and 21 and complementary sequences of said base sequences represented by SEQ ID NOS: 20 and 21, to be complementarily coupled with a seventh amplification product obtained by the PCR with the use of a fourth primer set which comprises a forward primer comprising a base sequence represented by SEQ ID NO: 8 and a reverse primer comprising a base sequence represented by SEQ ID NO: 11 as targets of amplification by the PCR for DNA of microorganisms belonging to the genus Pythium or Phytophthora and an ITS region of rDNA gene in DNA of microorganisms belonging to the genus Pythium or Phytophthora.

24. A carrier for detecting fungi **characterized by** immobilizing two or more probes selected from probe groups having base sequences enumerated in the following (a), (b) or (c):
(a) probes having base sequences represented by SEQ ID NOS: 101 to 106,
(b) probes which are hybridizable under stringent conditions with nucleic acid fragments comprising complementary sequences to base sequences represented by SEQ ID NOS: 101 to 106, and
(c) probes having base sequences complementary to the probes of (a) or (b).

25. The carrier for detecting fungi according to in claim 24 **characterized by** immobilizing a probe selected from each of the following groups of:
a first probe group comprising a probe, to detect Colletotrichum acutatum, having a base sequence represented by SEQ ID NO: 101 selected from an ITS region, and a probe having a base sequence represented by SEQ ID NO: 102 selected from β-tubulin genes,
a second probe group comprising a probe, to detect Fusarium solani, having a base sequence represented by SEQ ID NO: 103 selected from an ITS region, and a probe having a base sequence represented by SEQ ID NO: 104 selected from β-tubulin genes,
a third probe group comprising a probe, to detect Alternaria solani, having a base sequence represented by SEQ ID NO: 105 selected from an ITS region, and
a fourth probe group comprising a probe, to detect Rhizoctonia solani, having a base sequence represented by SEQ ID NO: 106 selected from an ITS region.

26. A method for detecting fungi which comprises a step amplifying a target region in DNA of the fungi of a detection target by PCR to confirm the presence or absence of an amplification product, the method for detecting fungi comprising:
including, in a sample, a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110,
in a case where at least one DNA of Colletotrichum acutatum, Fusarium solani, Alternaria solani and Rhizoctonia solani is contaned in the sample,
amplifying a target region in the DNA contained in the sample with the use of a reaction solution for the PCR, and
dropping a obtained amplification product on the carrier for fungi detection according to claim 24 or 25 to couple the obtained amplification product with a probe having a complementary base sequence.

27. A kit for detecting fungi comprising a primer set to amplify a target region in DNA of fungi as a detection target, and a carrier immobilizing a probe to confirm the presence or absence of an amplification product, wherein:
the primer set comprises:
a primer set to amplify an ITS region which comprises a forward primer comprising a base sequence represented by SEQ ID NO: 107 and a reverse primer comprising a base sequence represented by SEQ ID NO: 108, and a primer set to amplify a β-tubulin gene which comprises a forward primer comprising a base sequence represented by SEQ ID NO: 109 and a reverse primer comprising a base sequence represented by SEQ ID NO: 110, and
the carrier immobilizes:
a probe selected from each of the groups of:
a first probe group comprising a probe, to detect Colletotrichum acutatum, having a base sequence represented by SEQ ID NO: 101 selected from an ITS region, and a probe having a base sequence represented by SEQ ID NO: 102 selected from β-tubulin genes,
a second probe group comprising a probe, to detect Fusarium solani, having a base sequence represented by SEQ ID NO: 103 selected from an ITS region, and a probe having a base sequence represented by SEQ ID NO: 104 selected from β-tubulin genes,
a third probe group comprising a probe, to detect Alternaria solani, having a base sequence represented by SEQ ID NO: 105 selected from an ITS region, and
a fourth probe group comprising a probe, to detect Rhizoctonia solani, having a base sequence represented by SEQ ID NO: 106 selected from an ITS region.
